# EUROPEAN PATENT SPECIFICATION

(11) **EP 1 214 059 B1**
(45) Date of publication and mention of the grant of the patent: **25.05.2005**
(21) Application number: 00970467.7
(22) Date of filing: 21.09.2000
(51) Int. Cl.: A61K 9/14, A61K 9/107

(54) **SURFACE MODIFIED PARTICULATE COMPOSITIONS OF BIOLOGICALLY ACTIVE SUBSTANCES**
OBERFLÄCHENMODIFIZIERTE TEILCHENFÖRMIGE ZUSAMMENSETZUNGEN BIOLOGISCH AKTIVER STOFFE
COMPOSITIONS PARTICULAIRES, A SURFACE MODIFIEE, DE SUBSTANCES BIOLOGIQUEMENT ACTIVES

(30) Priority: 21.09.1999 US 154964 P
(43) Date of publication of application: 19.06.2002
(73) Proprietor: SKYEPHARMA CANADA INC., Verdun, Québec H3E 1H4 (CA)
(72) Inventor: PACE, Gary, W., Raleigh, NC 27615 (US); MISHRA, Awadhesh, K., Verdun, Quebec H3E 1HB (CA); SNOW, Robert, A., West Chester, PA 19380 (US)
(74) Representative: Bassil, Nicholas Charles
(86) International application number: PCT/US2000/025880
(87) International publication number: WO 2001/021154

(56) References cited:
- WO-A-00/10531
- WO-A-00/40219
- WO-A-00/41682
- WO-A-99/29300
- WO-A-99/29316
- WO-A-99/49848
- GB-A- 1 527 638
- US-A- 4 801 455
- US-A- 5 091 187
- US-A- 5 560 931
- US-A- 5 827 822
- US-A- 6 057 289

## Description

### BACKGROUND OF THE INVENTION

Oral, parenteral, and pulmonary delivery of biologically active substances, particularly water-insoluble pharmaceutically active substances, has been addressed by formulating these substances as micron and sub-micron sized particles suspended in water or ultimately present as a suspension in an aqueous environment. Various methods and compositions of such formulations have been reported in literature.

For instance, US Patents 5,091,188; 5,091,187 (D. H. Haynes, "Phospholipid-coated Microcrystals: Injectable Formulations of Water-Insoluble Drugs." Feb. 25, 1992) describe a MicroCrystal technology of delivering undiluted or highly concentrated drug substances as aqueous suspensions of micron- or submicron-sized particles stabilized with physiologically safe, tissue compatible, and pharmaceutically acceptable surface modifiers such as natural and synthetic bipolar lipids. Water-insoluble drugs are rendered injectable by formulation as aqueous suspensions of phospholipid-coated microcrystals. The crystalline drug is reduced to 50 nanometer to 10 micrometer dimensions by sonication or other processes inducing high shear in the presence of phospholipid or other membrane-forming amphipathic lipid. The membrane-forming lipid stabilizes the microcrystal by both hydrophobic and hydrophilic interactions, coating and enveloping it and thus protecting it from coalescence, and rendering the drug substance in solid form less irritating to tissue. Additional protection against coalescence is obtained by a secondary coating by additional membrane-forming lipid in vesicular form associated with and surrounding but not enveloping the lipid-encapsulated drug particles. Tissue-compatible formulations containing drug in concentrations up to 40% (w/v) are described. The preparations can be injected intra-lesionally and in numerous other sites, including intra-venous, intra-arterial, intra-muscular, intra-dermal, etc. The disclosure describes examples of formulations and pharmacokinetic data with antibiotics, anthelmintic drugs, anti-inflammatory drugs, local and general anesthetics, and biologicals. These compositions can be dried and reconstituted into aqueous suspension.

US Patent 5,145,684 describes aqueous suspensions of sub-micron size particles of poorly water-soluble therapeutic or diagnostic agent onto which are adsorbed a non-crosslinked surface modifier.

US patent 5,922,355 discloses aqueous suspensions of surface-modified submicron size particles of pharmaceutical or other water-insoluble or poorly water-soluble substances prepared using a combination of one or more surfactants such as polaxomers, poloxamines, polyoxyethylene sorbitan fatty acid esters and the like together with natural or synthetic phospholipids. The combination of phospholipids and surfactants allows the formation and stabilization of the sub-micron and micron size surface-modified particles in the form of their aqueous suspensions via hydrophilic, lipophilic and electrostatic interactions and therefore prevent these particles from aggregation or flocculation or particle size growth on storage. These aqueous suspensions of water-insoluble or poorly soluble compounds are manufactured in presence of the surface-modifying agents by a particle size reduction process such as sonication, homogenization, milling, microfluidization and precipitation, or recrystallization and precipitation of the compound using antisolvent and solvent precipitation.

The international publication WO 030615A1 teaches the use of careful selection of surface modifying agents on the basis of their hydrophile-lipophile balance (HLB) to prepare sub-micron and micron-size stable particles of water-insoluble or poorly soluble drugs or other industrially useful insoluble compounds suspended in an aqueous medium containing at least one surface modifier. The surface modifier or modifiers are selected by an HLB-based selection criteria for selecting the type and amount of surface modifiers used to obtain surface-modified sub-micron size stable aqueous suspensions of water insoluble or poorly water-soluble solid drugs.

One of the disadvantages of these technologies is that the surface modified particles of water-insoluble drug substances in aqueous suspension may tend to grow over time because of the dissolution and recrystallization phenomenon known as "Ostwald ripening".

Another disadvantage of these technologies is the requirement of removing water from the surface-modified particle suspensions prior to converting them to suitable solid dosage forms. It is known in the art of aqueous suspensions of surface-modified particles that in many instances water removal processes such as freeze drying and spray drying may cause excessive growth of the particle size or irreversible agglomeration and thereby adversely affect the advantageous properties of the microparticulate composition.

In addition, there are be applications where an aqueous medium is not suitable for surface-modified sub-micron particle suspensions. Alternative medium for such particle delivery systems can be a suitable oil or a suitable hydrophobic liquid or a suitable amphipathic medium in which the substance is substantially insoluble.

Various inventions have been disclosed relating to the suspension of surface modified particles of water-insoluble drugs in non-aqueous media or dispersed as an aqueous suspension within non-aqueous media.

For instance US Patents 5,091,188 and 5,091,187 claim that phospholipid surface modified particles prepared in aqueous suspension and then dried can be dispersed in a pharmacologically acceptable, water-miscible polar organic liquid with a dielectric constant greater than 30, which polar organic liquid does not substantially dissolve the lipid membrane or membranes or the drug substance or a mixture of the polar organic liquid with water. Additionally, a water-immiscible oil can be utilized to facilitate contact between the drug crystals or particles and the primary layer of amphipathic membrane-forming lipid, or to slow the rate of drug dissolution or to otherwise modify the rate of drug release. Also, within their aqueous suspension, the drug microparticles can be precoated by a layer of a waxy phospholipid-compatible solid having a melting point between 37 degree C and 100 degree C before or during the application of the primary layer of membrane-forming amphipathic lipid, the waxy phospholipid-compatible solid selected from paraffin, tristearin, ethyl oleate, cetostearyl alcohol, cetyl alcohol, myristyl alcohol, stearyl alcohol and petrolatum.

US patent 6,086,376 teaches the production of dry suspension aerosol formulation having a density in the range of from 1.0 to 1.5 g/ml consisting essentially of surface stabilized particles of water-insoluble drugs with a mean size range of 0.1 to 10 microns. The surface modifiers consist of one or more membrane-forming phospholipids and at least one surfactant surrounding the drug core. The surface modified particles are dispersed in 1,1,1,2-tetrafluoroethane HFA134a or 1,1,1,2,3,3,3-heptafluoropropane HFA 227 propellant, wherein the density of the coated drug microparticles substantially matches the density of the propellant, and the amount of phospholipid coating on the drug microparticles is more than 0.1% and less than 200% of the weight of the drug. Densities of various components of this invention are carefully controlled to provide a suspension of the surface modified drug microcrystals that upon agitation remains homogeneous for sufficiently long time to allow delivery of the said aerosol formulation. Even though water-free compositions of surface-modified drug microcrystals are suspended in pressurized fluorocarbon propellent, these aerosolizable compositions are neither intended to undergo self-dispersion upon exposing to aqueous environment, nor is there any requirement of exposing these pressurized propellant based compositions to aqueous medium prior to or during the intended drug delivery mode, i.e., inhalation of the aerosol.

Further, in US patents 5,560,931 and 5,571,536 is described formulations of water insoluble drug compounds as surface modified particles suspended in the aqueous phase of water-in-oil emulsions using digestible oils or fatty acids as the non-aqueous oil phase. The particles are formed by media milling in the presence of the surface modifier. The water (containing surface modified particles)-in-oil emulsion is then formed using a high shear emulsification procedure. The drug must be both water-insoluble and oil-insoluble. Drug insolubility in oil is required; otherwise it will partition into the oil phase by migrating from the water pool to the continuous oil phase and thus will render the formulation out of the scope of invention. This delivery system also suffers from the problem of presence of water that may not allow the use of many popular packaging systems such as hard- or soft-gelatin capsules or starch capsules, because these packaging systems are known to dissolve in aqueous media or their stability is severely compromised by even trace quantities of water. In addition, this technique may be suitable only for high potency drugs because of availability of a very small quantity of water in the oil-in-water emulsion which is the final medium of drug-delivery in this case.

In another method US patent 5,858,398 describes the formation of the particles of both water-soluble and insoluble drugs in the presence of a surfactant and suspending the particles in a micelle form in an oily substance. The microparticles described in this invention contain:
(1) at least one pharmaceutically-active agent, and
(2) at least one water soluble or miscible phospholipid, and
(3) at least one lipid soluble or miscible phospholipid, and
(4) at least one non-ionic surfactant having an HLB value of about 15 or greater, and
(5) at least one non-ionic surfactant having an HLB value of about or less, and
(6) at least one water soluble or miscible sterol compound.

The microparticles of the above composition are suspended in at least one fatty acid having a chain length of C14 or less. The composition may optionally contain at least one fatty acid having a chain length of C16 or greater in a concentration of about 5 w/v % or less.

The composition is prepared by admixing the pharmaceutically-active agent, phospholipids, surfactants, and sterol, micronizing the admixture to form microparticles, and suspending the microparticles in at least one fatty acid of chain length of C14 or less to form microparticles in a micelle. This invention is not designed to specifically address the problems associated with the formulation of water-insoluble drugs, especially stability and potential drug particle growth on exposure to water.

US patent 4,622,219 and US patent 4, 725, 442 describe a micron or sub-micron sized particulate composition of water-insoluble oily drug that is dispersed as an oil-in-water emulsion stabilized by emulsifiers such as natural or semi-synthetic phospholipids.

In another approach to the delivery of water-insoluble drugs, the drug may be dissolved in biocompatible oils. Further these solutions of the drug in oil may be dispersed in water using surfactants to form oil in water emulsions. A high-energy process such as a high-shear emulsification procedure is used to produce oil-in-water emulsions. These compositions require for their formation typically more than 70% of water. For example, US patent 5,660,858 describes pharmaceutical compositions consisting essentially of an oil-in-water emulsion containing a synthetic medium chain triglyceride in which is dissolved a therapeutically effective amount of a cyclosporin, phospholipid, and optionally free fatty acid or a salt thereof, non-ionic surfactant, ionic surfactant, glycerol, salts, buffers, preservative, osmotic modifier and antioxidant.

A special embodiment of oil-in-water type emulsions as carriers of drug substances is so-called solid lipid nanoparticles (SLN) which are prepared by using oils that melt at a temperatures higher than ambient temperature. The liquid oil-in-water emulsion is first made at a suitable temperature and then is cooled down to allow solidification of the oily core of the emulsion droplet. See Siekmann *et al*., Pharm. Pharmacol Lett., ***1***, 123-126 (1992) and Muller *et al*., European patent no. 0,605,497 (1996).

Oil-in-water emulsions are limited by the following:
(i) Solubility of the pharmaceutically or biologically active substance in oil or hydrophobic agent may not be enough that a solution can be prepared for making an oil-in-water emulsion using a preferably small quantity of oil or other hydrophobic agents.
(ii) A large volume of the dispersion (either oil-in-water or water-in-oil types) is necessary for delivering the required amount of the biologically active substance. Such quantities of dispersions cannot be packaged in convenient manner, for example, in hard- or soft-gelatin capsules, or in other types of capsules, or in coated tablets or pills.
(iii) Further, presence of water in these dispersions may not allow the use of many packaging systems such as hard- or soft-gelatin capsules or starch capsules because these packaging systems are known to dissolve in aqueous media.
(iv) In many cases the oil-in-water emulsions in the form of solid lipid nanoparticles are known to suffer from partial expulsion of the hydrophobic drug during a cooling step of their preparation which adversely affects the entrapment efficiency of the carrier system.

The formulation of both water-soluble and insoluble drugs in oil in water emulsions where the drug is dispersible or soluble in the oil has been applied to achieve sustained release of the drug.

In this regard the publication WO 041682A1 describes a lipophilic microparticle having an average particle size ranging from 0.1 to 200 micrometers, comprising a lipophilic substance and an active ingredient selected from the group consisting of a protein or peptide drug and an antigen, retains the full activity of the active ingredient, and when formulated in the form of an oil dispersion or oil-in-water emulsion, it releases in an in vivo environment the active ingredient in a controlled manner over a long period.

In another approach to the formulation of water-insoluble drugs the drug may be dissolved in water miscible organic solvents.

For example, US patent 6,046,163 describes therapeutic compositions comprising from 0.1 to 20% by weight of a water-insoluble cyclosporin and a vehicle comprising from 1 to 60% by weight of polyethers constituted by, preferably, substances like the water miscible ethoxy diglycol or polyethylene glycols 300 to 600 and/or substances like preferably dimethyl isosorbide, dimethyl isoidide and dimethyl isomannide, and from 1 to 60% by weight of a mixture of glyceryl monoesters of C8-C22 fatty acids and hexaglyceryl to pentadecaglyceryl monoesters of C8-C22 fatty acids in a ratio of 1:2 to 1:6.

In another approach the water-insoluble drug is dissolved in a mixture of oils and surfactants such that on addition to water the components spontaneously emulsify or disperse to form small particles of the oily phase in which the drug is dissolved. These small oil-phase particles are known as emulsion or microemulsion droplets. Microemulsions, also known as self-dispersing systems, differ from emulsions (which are heterogeneous dispersions of a discontinuous phase of droplets in a continuous phase of bulk liquid) by being as "a system of water, oil, and amphiphile which is a single optically isotropic and thermodynamically stable liquid solution" (see Danielsson and Lindman, "The definition of microemulsion" *Colloids Surf. **3**:* 391 (1981)). Oil-in-water type microemulsions are known to form spontaneously, without addition of energy, from microemulsion preconcentrates by mixing with aqueous component. Microemulsion (or emulsion) preconcentrates are compositions of the entire oil-in-water type microemulsion (or emulsion) components except water packaged in a convenient dosage form such as a dilutable and/or ingestable solution or capsule or gelcap.

In US patent 5,932,243 Fricker *et al*. teach about a pharmaceutical composition containing macrolide, e.g. a rapamycin compound in an emulsion preconcentrate or microemulsion preconcentrate for oral administration. The carrier medium for the rapamycin compound includes a hydrophilic phase, a lipophilic phase and a surfactant. The composition is stable and provides high absorption efficiency. The hydrophilic component, which, with co-components, if present, comprises 10 to 50% by weight of the carrier medium and which is Transcutol, Glycofurol, 1,2-propylene glycol, or mixtures thereof. The lipophilic component, which comprises 10 to 85% by weight of the carrier medium and which is selected from the group consisting of 1) fatty acid triglycerides, 2) mixed mono-, di-,and tri-glycerides, and 3) transesterified ethoxylated vegetable oils. The surfactant, which comprises 5 to 80% by weight of the carrier medium and which is selected from the group consisting of polyethyleneglycol, natural or hydrogenated castor oils, polyethylene-sorbitan fatty acid esters, polyoxyethylene fatty acid esters, polyoxyethylene-polyoxypropylene co-polymers, and block co-polymers. The relative proportion of the active ingredient and components is selected such that on dilution with water, a microemulsion having an average particle size of less than 150 nanometer is spontaneously formed.

Mulye teaches in US patent 6,057,289 about a self-emulsifying pharmaceutical compositions containing an effective amount of cyclosporin in association with a pharmaceutical carrier comprising (a) a cyclosporin solubilizing agent consisting essentially of a solubilizing effective amount of a fatty acid of 6 to 22 carbon atoms, and (b) a non-ionic surfactant having an HLB value greater than 10, said non-ionic surfactant being present with the cyclosporin solubilizing agent and cyclosporin in an amount sufficient to form an emulsion when brought into contact with an aqueous medium in a mammal. Certain specific compositions may form microemulsions upon mixing with aqueous medium.

Similarly, Lacy et al, teach in US patent 6,096,338 (and in an earlier US patent 5,645,856) to prepare a carrier for hydrophobic drugs, and pharmaceutical compositions based thereon, which carrier comprises a digestible oil and a pharmaceutically acceptable surfactant component for dispersing the oil in vivo upon administration of the carrier, which comprises a hydrophilic surfactant, said surfactant component being such as not to substantially inhibit the in vivo lipolysis of the digestible oil.

The following should be noted about compositions of US patents 6,096,338 and 5,645,856:
(1) that this carrier system is suitable only for the hydrophobic drugs;
(2) that the carrier system is designed to facilitate only a digestible oil vehicle;
(3) on addition into aqueous medium this mixture disperses into an oil-in-water type emulsion;
(4) that this carrier system does not attempt to obtain a self-dispersible system that results in a micron or sub-micron size surface-modified and surface-stabilized solid particles of the active substance;
(5) that the surfactant system is designed to avoid inhibitory interference on the gastro-intestinal lipolysis of the oil, which is considered by the author as necessary for bioavailability improvement of the orally administered drug; and
(6) that the compositions of this patent are limited to only the peroral dosage forms.

WO 99/29300 A discloses a self-emulsifying fenofibrate composition comprising a surfactant, e.g., Tween 80/Myrj 52/Vitamin E-TPGS; a non-aqueous phase, e.g., Miglyol/linoleic acid; and a hydrophilic substance, e.g., ethanol/propylene glycol, and having submicron and micron average particle sizes. WO 99/29300 A does not teach or disclose a non-aqueous carrier system containing a phospholipid as a surfactant system that is soluble in non-aqueous medium comprising the composition.

WO 99/29316 A discloses microemulsions comprising cyclosporin, oil, a surfactant and a hydrophilic substance. WO 99/29316 A does not teach or disclose a non-aqueous carrier system containing a phospholipid as a surfactant system that is soluble in non-aqueous medium comprising the composition.

U.S. Patent No. 5,827,822 discloses a solution of cyclosporin, ethanol/propyleneglycol and surfactant (Tween 80), which forms microparticles in water. U.S. Patent No. 5,827,822 does not teach or disclose a non-aqueous carrier system containing a phospholipid as a surfactant system that is soluble in non-aqueous medium comprising the composition.

U.S. Patent No. 5,091,187 discloses phospholipid-coated microparticles of water insoluble drugs that can be formulated with Vitamin E oil. U.S. Patent No. 5,091,187 does not disclose a non-aqueous carrier system that provides stable, surface-modified micron or submicron dispersible particles of Applicant's particular biologically active agents. Unlike the present invention, the phospholipid coated microparticles in aqueous suspension as disclosed in U.S. Patent No. 5,091,187 are disadvantageous in that they tend to "grow" over time because of dissolution and recrystallization - a phenomenon known to the skilled practitioner as "Ostwald ripening".

U.S. Patent No. 5,560,931 discloses aqueous dispersions of amorphous nanoparticles of cyclosporin in a formulation comprising a lower alkanol co-solvent and a polyoxyalkylene surfactant. U.S. Patent No. 5,560,931 does not teach or disclose a non-aqueous carrier system containing a phospholipid as a surfactant system that is soluble in non-aqueous medium comprising the composition.
U.S. Patent No. 4,801,455 discloses dihydroxyanthraquinone finely ground in Miglyol, mixed with lecithin and emulsified in water for injection. U.S. Patent No. 4,801,455 does not disclose surface-modified microparticles containing Applicant's particular biologically active substances and dispersed in a non-aqueous carrier system comprising non-aqueous medium, phospholipid surfactant system and one or more hydrophilic substances.

GB Patent No. 1,527,638 discloses suspension formulations of niclosamide ground in oily liquid excipient, e.g., paraffin or vegetable oil. A surface active agent or wetting agent, e.g., lecithin, is optionally added. U.S. Patent No. 1,527,638 does not disclose surface-modified microparticles containing Applicant's particular biologically active substances and dispersed in a non-aqueous carrier system comprising non-aqueous medium, phospholipid surfactant system and one or more hydrophilic substances.
While it is not intended to present a comprehensive review of background materials, the above examples demonstrate nonexistence of compositions capable of delivering water-insoluble or poorly water-soluble biologically active substances (that may be either oil-soluble or oil-insoluble) via self-dispersible systems providing stable surface-modified micron or submicron size solid particles of the active agent. The examples mentioned as the background material, although related to the subject matter of the current inventive compositions and processes, they are different from the present invention.

### SUMMARY OF THE INVENTION

The invention is as defined in the claims.

This invention disclosure relates to compositions useful for the delivery of stable surface modified sub-micron and micron sized particles of water-insoluble biologically active substances from a carrier system comprising a non-aqueous medium(s) and surfactant(s) and optionally a hydrophilic component that self-disperses on exposure to an aqueous environment. In the aqueous environment the dispersed phase comprises components of the carrier system and the surface modified particles. Surface modified particles refers to micron or sub-micron size solid particles of a water-insoluble active ingredient that is surface-modified by the application of surface active agents to stabilize the said particles against particle size growth, flocculation, aggregation, or agglomeration. In the current invention, the stable surface-modified particulate dispersion of the water-insoluble biologically active substances may be formed in the carrier system using a high-energy dispersion technique such as homogenization or milling. Alternatively, the water-insoluble biologically active substances may be dissolved in a suitable carrier system and then on exposing this solution *in-vitro* or *in-vivo* to aqueous media a self-dispersion of surface modified particles of the water-insoluble drug and other components of the carrier medium is formed. The dispersed surface modified particles may be present in the dispersed non-aqueous medium or in the aqueous phase or both.

The term "water-insoluble biologically active substance" as used herein shall mean any water-insoluble or poorly water-soluble biologically active substance.

The term "non-aqueous composition" as used herein shall refer to any composition that is devoid of water.

The term "carrier system" as used herein is to be understood as defining the material in which the preparation of the stable water-insoluble biologically active substance of this invention is made. The carrier system comprises at least one component from (i) non-aqueous media in which a water-insoluble biological agent is soluble, or non-aqueous media in which a water-insoluble biological agent is insoluble or poorly soluble; and at least one component from (ii) a surfactant system; and optionally at least one component from (iii) a hydrophilic component.

The term "aqueous medium" is meant water; buffered water including phosphate buffered water, phosphate buffered saline, citrate buffered water, acetate buffered water, water buffered with pharmaceutically acceptable pH controlling agents; water containing salts such as sodium chloride and other pharmaceutically acceptable salts; water containing soluble agents for lyoprotection or cryoprotection such as dextrose, mannitol, trehalose, sucrose, sorbitol, and other pharmaceutically acceptable lyoprotectants and cryoprotectants; water containing soluble agents used to facilitate spray drying such as polyhydroxy-containing compounds such as sugars, polyols, and water containing mixtures of these buffers, agents and compounds. In one embodiment, the aqueous medium can contain one or more soluble surface active agent. In another embodiment, the aqueous medium can contain one or more surfactants dispersed such as by shear mixing into water or other aqueous medium as described herein. In yet another embodiment, the aqueous medium can comprise a biological fluid such as blood, plasma, saliva, urine, a protein-containing solution, an aqueous suspension of a protein, lymph fluid, semen, vaginal fluid, lachrymal fluid, nasal fluid, synovial fluid, cerebral fluid, cerebralspinal fluid, amniotic fluid, pancreatic fluid, pulminary fluid, ascites fluid, fluid from a cyst, gastric fluid, intestinal fluid, and other fluids that can be removed from a patients for use in this invention. Fluids from an individual patient are inherently biocompatible in most cases with that individual, and a suspension of this invention in a fluid from a patient can be administered to that patient as a biocompatible fluid suspension. Optionally, such patient derived biological fluids can be diluted, for example with other aqueous media as described herein. Optionally, such patient derived biological fluids can be concentrated by removal of some of the water contained therein. Optionally, such patient derived biological fluids can be mixed or blended with other biological fluids from the same patient or from different patients.

In preferred compositions of this invention the non-aqueous carrier system consists of at least one non-aqueous medium component, at least one surfactant component, and optionally at least one hydrophilic component.

Preferred non-aqueous media of the carrier system include hydrophobic components such as triglycerides, diglycerides, monoglycerides, free fatty acids, and fatty acid esters and derivatives thereof, individually or in combination. Examples of such hydrophobic components include but are not limited to propylene glycol dicaprylate/caprate, caprilic/capric triglyceride, caprylic/capric/linoleic triglyceride, e.g. synthetic medium chain triglycerides having C₈₋₁₂ fatty acid chains or other derivatized (synthetic) triglycerides of the type known and commercially available under Miglyol 810, 812, 818, 829 and 840, linoleic acid, linoleic acid ethyl ester, fish oils as free fatty acids, their esterification and their transesterification products, e.g. of the type known and commercially available under EPAX6000FA, EPAX4510TG, individually or in combination. Additional examples include vegetable oils and C₁₂₋₁₈ fatty acid mono-, di- and triglycerides prepared by individual admixing or as transesterification products of vegetable oils (such as soybean oil, almond oil, sunflower oil, olive oil or corn oil) with glycerol.

Additional non-aqueous media include pharmaceutically acceptable non-aqueous solvents in which biological substance is either soluble or insoluble or poorly soluble. These media may be derived from the classes of organic chemicals, such as, monohydric alcohols *e*.*g*., alkanols; dihydric alcohols *e*.*g*., glycols; polyhydroxy compounds *e*.*g*., glycerin; aromatic esters, *e*.*g*., benzyl benzoate, diethyl phthalate, propyl gallate, triacetin, diacetin, monoacetin, triethyl citrate; water soluble organic solvents such as propylene carbonate and glycofurol, dimethyl isosorbide, dimethyl isoidide and dimethyl isomannide.

One or more surfactants utilized in the carrier of this invention is termed as the surfactant system. In those compositions possessing more than one surface active agents, the principle surface active agent, that is present in larger quantity is called the surfactant and the other surface active agents are named as co-surfactants.

The surfactant system of this invention may include natural or synthetic surface modifying agents selected from phospholipids, and non-ionic, anionic or cationic surfactants or combinations thereof. These surface modifying molecules adhere onto the surfaces of the insoluble-particles of biologically active substances and stabilize the sub-micron and micron size drug particles. The stabilization of the particulate matter against aggregation, flocculation, or particle-size growth is thought to arise from lipophilic, amphipathic, and/or electrostatic interactions as well as steric hindrance. In addition, alteration of the density of the medium, the polarity and the surface tension of the particles suspended in the medium, and favorable adjustment of the hydrodynamic properties reduce the tendency of dispersed particles to either cream or sediment. Also, addition of the surface-active substances to these compositions enables them to easily disperse upon release into an aqueous environment such as a biological fluid, particularly gastric and gastro-intestinal fluids, blood, and lymph.

In the compositions of this invention a preferred surfactant system comprises at least one phospholipid selected from the group consisting of saturated phospholipids, unsaturated phospholipids, synthetic phospholipids, natural phospholipids, and combinations thereof.

The hydrophilic component when optionally used comprises less than about 10% of the carrier system. Examples of hydrophilic components include low-molecular weight monohydric alcohols and preferably ethanol, low-molecular weight polyhydric alcohols, glycols, and glycerol, and mixtures thereof. In a preferred embodiment, the hydrophilic component comprises a pharmaceutically acceptable monohydric or polyhydric alcohol.

A typical carrier system of this invention consists of one or more suitable hydrophobic oils and a surfactant system comprising at least one surface modifying agent and optionally one or more hydrophilic components. The composition of this invention may remain in a solid state or a semi-solid state such as waxy or gel state or in liquid state. In addition to the carrier system the composition of this invention may also preferably additionally comprise further adjuvants like anti-oxidants, preserving agents and stabilizers, flavoring agents, thickening agents, diluents and other pharmaceutically acceptable ingredients for other purposes. The compositions of this invention are highly concentrated and suitable for packaging as a capsule or tablet and thus are very convenient for use in oral dosage forms. Additionally, compositions of this invention may be used to effectively deliver biologically active substances via other routes of drug delivery, such as, but not limited to peroral, parenteral, transdermal, pulmonary (inhalation), ophthalmic, transmucosal routes or by injection.

### BRIEF DESCRIPTION OF THE FIGURES

Figure 1 is a schematic illustration of surface modified particles of water-insoluble substance in non-aqueous medium dispersed in aqueous medium.
Figure 2 is a process flowchart for obtaining micronized and surface stabilized biological substance dispersed in non-aqueous self-dispersing carrier medium having particles in a size range of about 0.01 to about 10 micrometer, and preferably 0.01 to 2 micrometer.
Figure 3 is a process flowchart for obtaining anti-solvent precipitated and surface stabilized biological substance dispersed in non-aqueous self-dispersing carrier medium having particles in a size range of about 0.01 to about 10 micrometer, and preferably 0.01 to 2 micrometer.
Figure 4 is a process flowchart for obtaining dispersion of surface stabilized biological substance in non-aqueous self-dispersing carrier medium having particles in a size range of about 0.01 to about 10 micrometer, and preferably 0.01 to 2 micrometer.
Figure 5 is a process flowchart for obtaining a solution of water-insoluble active substance in non-aqueous self-dispersing carrier medium.

### DESCRIPTION OF THE INVENTION

Surprisingly, it has been found that suspensions of stable surface modified micron and sub-micron sized particles in self-dispersing carrier systems are attainable. These particles consist of a water-insoluble biologically active substance coated with at least one surface-modifying agent, e.g., phospholipid molecules or other surface modifying agents or combinations thereof. Preferably the particles are coated with phospholipid(s).

In one embodiment of this invention particles of a biologically active water-insoluble or poorly soluble substance of about 0.01 to about 10 micrometer volume weighted mean particle size are stabilized with surface modifiers and dispersed in a non-aqueous medium in which the substance is insoluble or poorly soluble. The active substance may be released to an aqueous environment as surface modified submicron or micron sized particles of the water-insoluble substance from this composition.

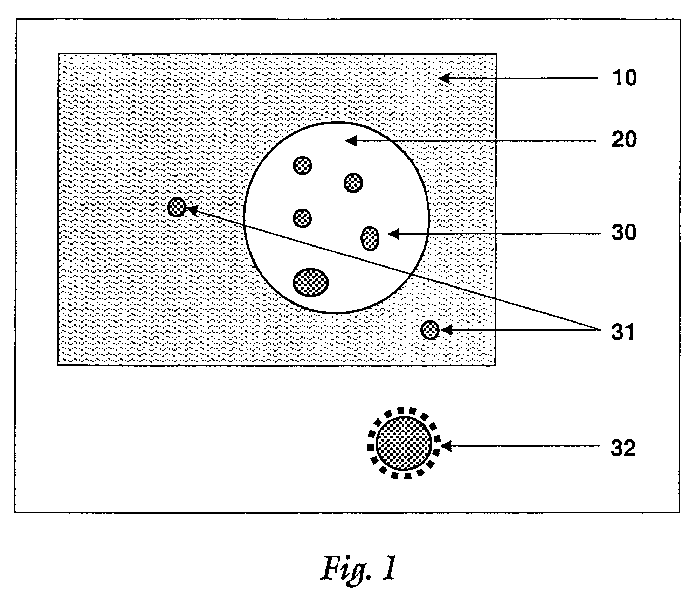

In another embodiment of this invention the above mentioned composition of the water-insoluble biologically active substance is added to an aqueous medium, for example, the fluid medium of an *in-vivo* environment, and self-disperses to form non-aqueous droplets and surface modified particles (see Figure 1). The active substance may be released to the *in-vivo* environment from this self-dispersion.

Figure 1 is a schematic illustration of surface modified particles of water-insoluble substance in non-aqueous medium dispersed in aqueous medium. Item 10 represents the aqueous environment or aqueous medium. Item 20 represents the self-dispersed non-aqueous medium droplet. Item 30 represents stable surface modified particles of water-insoluble or poorly water-soluble biologically active substance. Item 31 represents surface modified particles of water-insoluble or poorly water-soluble biologically active substance migrated into the aqueous medium. Item 32 is a magnified schematic illustration of the surface modified particles of water-insoluble or poorly water-soluble biologically active substance.

In another embodiment of this invention, there is provided composition comprising stable particles of a surface modified water-insoluble biologically active substance of a mean size in the range of 0.01 to 10 micrometers, which particles are dispersed in a non-aqueous carrier system comprised of a non-aqueous medium in which the biologically active substance is not soluble or is poorly soluble; and a surfactant system consisting of at least one surfactant which is soluble in the non-aqueous medium and which absorbs to the surface of the biologically active substance; and optionally a quantity of not more than about 10% of the total weight of said composition of one or more hydrophilic substance that provides a self-dispersing property to said composition wherein said composition can self-disperse upon addition to an aqueous medium to form a suspension of components of the non aqueous carrier system and stable particles of said water-insoluble biologically active substance and wherein said particles have a size in the range of 0.01 to 10 micrometer and have associated therewith on the surface at least a portion of said surfactant system.

In yet another embodiment of the invention there is provided composition comprising a water-insoluble biologically active substance mixed with a non-aqueous carrier system, wherein said carrier system comprises a non-aqueous medium in which said water-insoluble biologically active substance is soluble; and a surfactant system consisting of at least one surfactant; and optionally a quantity of not more than about 10% of the total weight of said composition of one or more hydrophilic substance that provides a self-dispersing property to said composition; wherein said composition can self-disperse upon addition to an aqueous medium to form a suspension comprising components of the non-aqueous carrier system and stable particles of said water-insoluble biologically active substance wherein said particles have a mean size in the range of 0.01 to 10 micrometer and have associated therewith on the surface at least a portion of said surfactant system.

Surface modifiers are selected such that they adsorb to the surface of the water-insoluble substance when exposed to the non-aqueous carrier and when dispersed in aqueous media to allow the formation of stable small particles of the substance (Figure 1).

In yet another embodiment of the invention, the carrier system consists of non-aqueous medium and a surfactant system in which a biologically active substance is dissolved, such that upon mixing with an aqueous environment the composition self-disperses to form an oil-in-water dispersion in which the droplet size is sufficient to entrap one or more particles of biologically active substance. Alternatively, upon mixing with an aqueous environment the composition may self-disperse to form surface modified micron and submicron sized particles of the water-insoluble substance wherein the particles are at least in-part external to the non-aqueous carrier system.

Such novel compositions provide preferred dosage forms of pharmaceutical agents useful to achieve certain pharmacological actions such as controlled and/or sustained delivery of a drug substance, targeted delivery of a drug substance such as to the lymphatic system. They also can provide a dosage form with reduced tissue irritation such as commonly found after oral delivery of non-steroidal anti-inflammatory drugs.

In addition, there are applications where aqueous medium may not be suitable for the surface modified sub-micron particulate suspensions. Presence of water in these suspensions may not allow the use of certain packaging systems such as hard- or soft-gelatin capsules or starch capsules because these packaging systems are known to dissolve in aqueous media. Preferred medium for such non-aqueous particulate suspensions or dispersions may be a suitable oil or a suitable hydrophobic liquid or a suitable amphipathic medium to allow a traditional packaging system, such as a tablet, gelcap, or capsule to be employed.

### DESCRIPTION OF THE PREFERRED EMBODIMENTS

The present invention can be practiced with a wide variety of water-insoluble biologically active substances that are either insoluble or poorly soluble in hydrophobic liquids. One embodiment of this invention may utilize even those water-insoluble biologically active substances that are soluble in non-aqueous media.

Examples of some preferred biologically active agents useful in this invention include antifungal agents, immunosuppressive and immunoactive agents, antiviral agents, antineoplastic agents, analgesic and antiinflammatory agents, antibiotics, antiepileptics, anesthetics, hypnotics, sedatives, antipsychotic agents, neuroleptic agents, antidepressants, anxiolytics, anticonvulsant agents, antagonists, neuron blocking agents, anticholinergic and cholinomimetic agents, antimuscarinic and muscarinic agents, antiadrenergic and, antarrhythmics, antihypertensive agents, antineoplastic agents, hormones, nutrients, oligonucleotides, vaccine, peptides or peptidomimetics and proteins. A detailed description of these drugs may be found in *Remington's Pharmaceutical Sciences,* 18th Edition, 1990, Mack Publishing Co., PA.

More preferred biologically active substances include nifedipine, alfaxalone, budesonide, a hormone, ursadiol, propanolol, itraconazole, acyclovir, fenofibrate and their analog derivatives. Potential imaging agents include water-insoluble X-ray or Magnetic Resonance Imaging nanoparticulate material.

In one aspect, preferred biologically active substances members of the group consisting of an antihypertensive drug; nifedipine; an anticholinergic drug; ursodiol; a drug for treating a gastro-intestinal disorder; budesonide; an antineoplastic drug; peclitaxel; camptothecin; a derivative of peclitaxel; a derivative of camptothecin; an NSAID; piroxicam; an anti-fungal agent; itraconazole; an anti-viral agent; acyclovir; a derivative of acyclovir; a cholesterol controlling agent; fenofibrate; an immuno-suppressive peptide; cyclosporine; a protein used in the treatment of diabetes; insulin; and a derivative of insulin.

Preferred non-aqueous media of the carrier system include hydrophobic components such as triglycerides, diglycerides, monoglycerides, saturated or unsaturated free fatty acids, mixtures of saturated and unsaturated free fatty acids, and fatty acid esters and derivatives thereof, individually or in combination. Examples of such hydrophobic components include but are not limited to propylene glycol dicaprylate/caprate, caprilic/capric triglyceride, caprylic/capric/linoleic triglyceride, e.g. synthetic medium chain triglycerides having C₈₋₁₂ fatty acid chains or other derivatized (synthetic) triglycerides of the type known and commercially available under Miglyol 810, 812, 818, 829 and 840, as well as linoleic acid, linoleic acid ethyl ester, fish oils as free fatty acids, their esterification and their transesterification products, e.g. of the type known and commercially available under EPAX6000FA, EPAX4510TG, all individually or in combination. Additional examples include vegetable oils and C₁₂₋₁₈ fatty acid mono-, di- and triglycerides prepared by individual admixing or as transesterification products of vegetable oils (such as soybean oil, almond oil, sunflower oil, olive oil or corn oil) with glycerol.

Additional non-aqueous media include pharmaceutically acceptable non-aqueous media or solvents in which the biological substance is either soluble or insoluble or poorly soluble. These solvents may be selected from classes of organic chemicals such as but not limited to, monohydric alcohols e.g., alkanols; dihydric alcohols e.g., glycols; polyhydroxy compounds e.g., glycerin; aromatic esters, e.g., benzyl benzoate, diethyl phthalate, propyl gallate; nonaromatic esters such as triacetin, diacetin, monoacetin, triethyl citrate; water soluble organic solvents such as propylene carbonate and glycofurol, dimethyl isosorbide, dimethyl isoidide and dimethyl isomannide.

Surface modifiers or their combinations are selected from known organic and inorganic excipients suitable for the delivery of the biologically active substance such as pharmaceutical excipients. Such excipients include various pharmaceutically useful polymers, low molecular weight oligomers, natural products and surfactants. Preferred surface modifiers include nonionic and ionic surfactants. Examples of some suitable surface modifiers are:
(a) natural or synthetic lipophilic agents , *e.g.*, phospholipids, cholesterol, and cholesteryl fatty acid esters and their surface active derivatives;
(b) nonionic surfactants such as polyoxyethylene fatty alcohol ethers, sorbitan fatty acid esters, polyoxyethylene sorbitan fatty acid esters (Tweens), sorbitan esters, glycerol esters such as Myrj and glycerol triacetate (triacetin), polyethylene glycols, cetyl alcohol, cetostearyl alcohol, stearyl alcohol, poloxamers, polaxamines, polyoxethylene castor oil derivatives (Cremophors), vitamin E or its derivatives, such as D-a-tocopheryl polyethylene glycol 1000 succinate (vitamin E TPGS), PEG glyceryl fatty acid esters such as PEG-8 glyceryl caprylate/caprate (commercially known as Labrasol), PEG-4 glyceryl caprylate/caprate (Labrafac Hydro WL 1219), PEG-32 glyceryl laurate (Gelucire 44/14), ), PEG-6 glyceryl mono oleate (Labrafil M 1944 CS), PEG-6 glyceryl linoleate (Labrafil M 2125 CS), propylene glycol mono- and di-fatty acid esters, such as propylene glycol laurate, propylene glycol caprylate/ caprate; also diethylene glycol monoethyl ether, commercially known as transcutol, sorbitan fatty acid esters, such as the type known and commercially available under the trade name Span (e.g., Span 20); monoglycerides and acetylated monoglycerides, e.g., glycerol monooleate, glycerol monostearate and mono-and di-acetylated monoglycerides, monoacetin, diacetin; carbomers, such as the type known and commercially available under the trade name Carbopol;
(c) anionic surfactants such as bile salts, potassium laurate, triethanolamine stearate, sodium lauryl sulfate, alkyl polyoxyethylene sulfates, sodium alginate, dioctyl sodium sulfosuccinate, sodium carboxymethylcellulose, and calcium carboxymethylcellulose;
(d) cationic surfactants such as quaternary ammonium compounds, benzalkonium chloride, cetyltrimethylammonium bromide, and lauryldimethylbenzyl-ammonium chloride;
(e) substituted cellulose derivatives such as methylcellulose, hydroxycellulose, hydroxy propylcellulose, hydroxy propylmethylcellulose, noncrystalline cellulose, sodium carboxymethyl cellulose;
(f) polyethylene glycol (PEG); e.g. PEG 300, PEG 400, PEG 600, PEG 1000, PEG 1500, PEG 3400; such as the type known and commercially available under the trade name Carbowax, Lutrol E and Hodag PEG;
(g) colloidal clays such as bentonite and veegum;
(h) natural proteins such as casein, gelatin;
(i) trgacanth, waxes, enteric resins, paraffin, acacia.

The non-aqueous hydrophobic media are selected from triglycerides, oils derived from vegetable or animal origins such as vegetable oils or fish oils, free fatty acids such as oleic or linoleic acids or other poly-unsaturated fatty acids, fatty acid esters, and other pharmaceutically suitable hydrophobic compounds that remain in liquid state at ambient temperature and pressure such as hydrofluorocarbons such as perflubron.

Optionally, small quantity of low-molecular weight hydrophilic substances, for example, monohydric or polyhydric alcohols, such as ethanol or glycols or glycerol may be also added.

A detailed description of these surface modifying agents and lipophilic or hydrophobic media may be found in *Remington's Pharmaceutical Sciences,* 18th Edition, 1990, Mack Publishing Co., PA; and *Theory and Practice of Industrial Pharmacy*, Lachman *et al*., 1986.

In compositions of this invention possessing more than one surface active agent the principle surface active agent, *i.e.*, that present in largest quantity is called the surfactant and the other surface active agents are called as co-surfactants. When present in equal amounts, the surfactant is defined by the higher molar amount if w/w ratio is used or the higher weight amount if molar ratio is used.

The type and amount of surfactant and co-surfactant used is based on the relative solubility and/or polarity of these ingredients. The formulation compositions are hence optimized with respect to each biologically active agent individually. It is thought that some of the functions of the surface modifier(s) as it relates to this invention are:
- to suppress the process of Ostwald Ripening and therefore maintain the particle size, increase the storage stability, minimize sedimentation, and decrease the particle growth during lyophilization and other unit-processes of the manufacture of these compositions;
- adhere or coat firmly onto the surfaces of particles and therefore modify the interfaces between the particles and the non-aqueous carrier system and the aqueous environment when self-dispersion occurs;
- increase the interface compatibility between particles and the non-aqueous carrier system and the aqueous environment when self-dispersion occurs; and
- facilitate self-emulsification or self-dispersion capability of the composition.

### METHODS

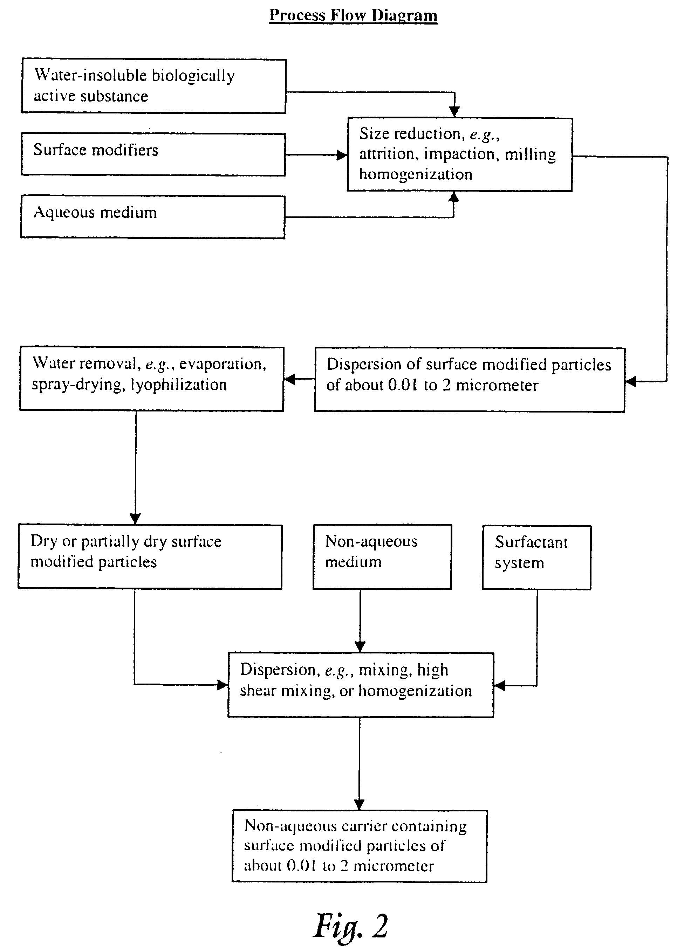

A dispersion of particles of a biologically active substance in a non-aqueous carrier system in which it is insoluble or poorly soluble is formed using one of the following four processes. These processes are schematically outlined in Figures 2-5.

### Process I: Micronized surface stabilized water-insoluble biological substance dispersed in a non-aqueous carrier system.

The first of these processes (Figure 2) utilizes a particle size reduction unit process to form aqueous suspensions of surface modified sub-micron or micron sized particles of the water-insoluble substances that are also insoluble in the non-aqueous carrier system. In this process an aqueous medium premix of the water-insoluble biologically active substance and the surfactant system is prepared and then subjected to one of the particle size reduction unit processes. A suspension of surface modified small particles, ranging from 0.01 to 10 micrometer volume weighted mean particle size is obtained. This suspension is subjected to a drying unit process such as spray drying or lyophilization yielding a dry powder of the particles, which is then mixed or homogenized with or without high shear with non-aqueous carrier system, such as oily medium, and optionally with additional components of the surfactant system. The dispersion of the particles in the self-dispersing non-aqueous carrier so obtained is then available for final packaging.

### Process II: Anti-solvent precipitated surface stabilized water-insoluble biological substance dispersed in non-aqueous carrier.

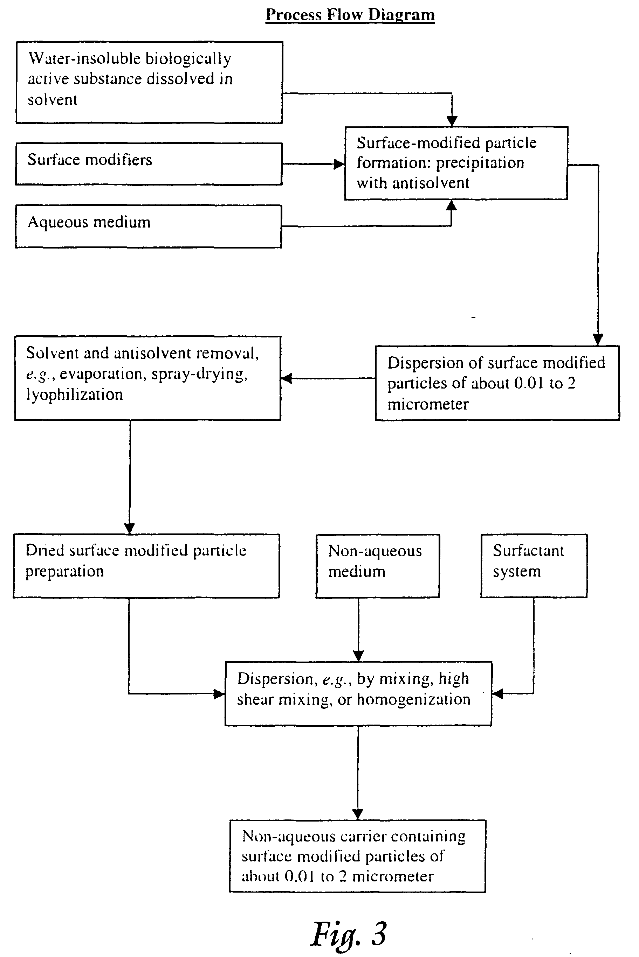

In the second process (Figure 3) the water-insoluble biologically active substance is dissolved in appropriate organic solvent. Surface modifier of the surfactant system is mixed either in this solution or in an antisolvent for the biologically active substance, which can be an aqueous medium. The antisolvent medium and the organic solution with the biologically active substance are mixed to cause the antisolvent precipitation of the biologically active substance. If required, this dispersion is further subjected to a size reduction unit process (described below). A suspension of surface modified small particles, ranging from 0.01 to 10 micrometer volume weighted mean particle size is obtained. This suspension is subjected to a solvent and antisolvent removal unit process such as spray drying or lyophilization yielding a dry powder of the surface modified particulate composition, which is then mixed or homogenized with or without high shear with the non-aqueous carrier system, such as the oily medium, and optionally with additional components of the surfactant system. The non-aqueous dispersion of the microparticulate suspension so obtained is then available for final packaging.

### Process III: Dispersion of surface stabilized water-insoluble biological substance in non-aqueous self-emulsifying carrier system.

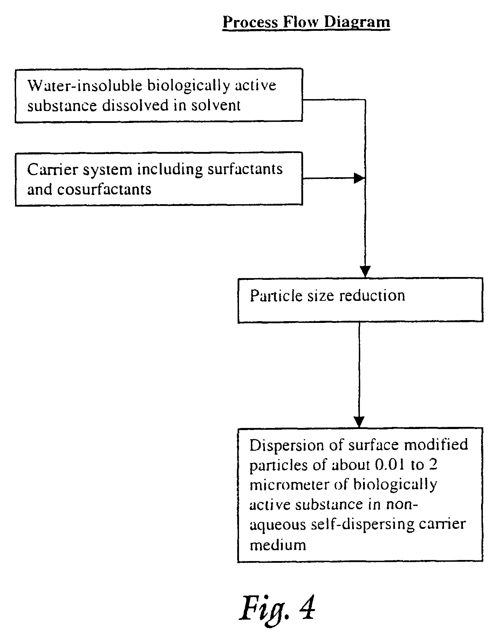

In the third process (Figure 4) is performed a particle size reduction unit process (see below) on the premix of water-insoluble biologically active substance and the carrier system to directly produce the suspensions of the active substances that are insoluble or poorly-soluble in the carrier system. The non-aqueous dispersion of the particles in the self-dispersing non-aqueous carrier system so obtained is then available for final packaging.

In this case, the composition is selected such that upon addition of aqueous medium these compositions self-disperse. The droplets of the self-dispersion contain particles of the surface stabilized water-insoluble biological substance suspended in the oily droplets of the dispersion.

### Process IV: Dispersion of solution surface stabilized water-insoluble biological substance in non-aqueous self-emulsifying medium.

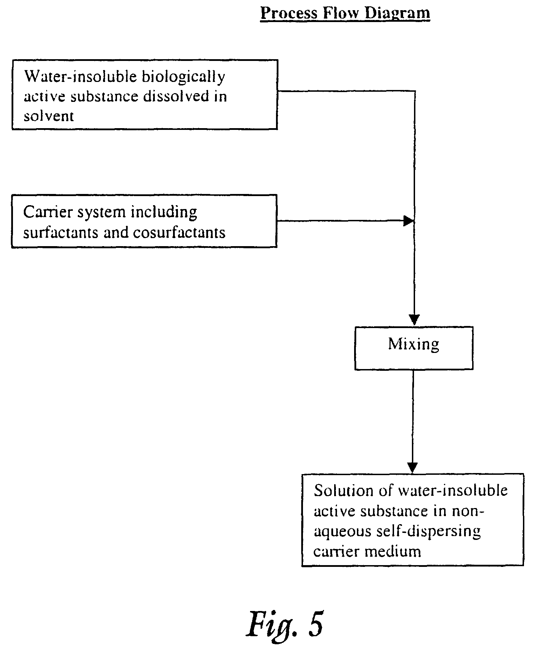

The fourth process (Figure 5) relies on making compositions of the substances that are soluble in the carrier system such that upon addition of aqueous medium these compositions self-disperse into sub-micron and micron sized particles of surface stabilized water-insoluble biological substance. The non-aqueous mixture of the biologically active substance so obtained is then available for final packaging.

In this case, the composition is selected such that upon addition of aqueous medium these compositions self-disperse. The droplets of the self-dispersion may contain the surface modified particles of the water-insoluble biologically active substance suspended in the oily droplets of the dispersion. Alternatively, the self-dispersion process may provide a particle suspension of the active substance wherein the components of the carrier system adhere to the small active substance particle to facilitate spontaneous formation of the said dispersed surface modified particles.

### Particle Size Reduction Unit Processes:

The particles must be reduced in size at a temperature that does not significantly degrade the drug substance. Processing temperatures of less than about 30-40 degree C are usually preferred. If required, the processing equipment and the process-fluid flow is cooled with conventional cooling equipment. The method is conveniently carried out under conditions of ambient temperature. When size reduction is carried out in aqueous medium, pH is a very important parameter to control. It is controlled within a range so that the active and the inactive ingredients do not undergo pH-induced degradation. Similarly for the compounds sensitive to oxidation proper nitrogen blanketing procedure is applied.

***High Energy Micronization:*** The biologically active substance together with other appropriate ingredients are subjected to high energy provided by high pressure homogenization or microfluidization as known in the art. In the these processes, the high energy results in shear, impaction, cavitation and other forces that causes particle size reduction.

### Media Milling

The particle size reduction of the drug substance can achieved by a dispersion mill. Suitable dispersion mills include a ball mill, an attritor mill, a vibratory mill, and media mills such as a sand mill and a bead mill. The grinding media for the particle size reduction step can be selected from rigid media preferably spherical or particulate in form having an average size less than about 3 mm and, more preferably, less than about 1 mm. The selection of material for the grinding media is not believed to be critical. We have found that zirconium oxide, such as zirconia stabilized with magnesia, zirconium silicate, stainless steel, titania, alumina, zirconia stabilized with yttrium and glass grinding media provide particles having minimal levels of contamination which are believed to be acceptable for the preparation of pharmaceutical compositions.

### Rapid Expansion of a Supercritical Solution

The biologically active substance is dissolved in a supercrtical gas such as carbon dioxide that may also contain the surface modifiers and then the solution is expanded over an aqueous suspension with or without the surface modifiers. The principle feature of this unit process is rapid attainment of intimate contact of the dissolved drug and the surface modifier during the very fast precipitation step of the drug from their solution in the liquefied gas. While very rapid precipitation is a characteristic of precipitation of solutes from supercritical fluids, the rapid intimate contact with the surface modifier is achieved by having the surface modifiers dissolved in the liquefied-gas containing the dissolved drug. A rapid intimate contact between the surface modifier and the newly formed particle substantially inhibits the crystal growth of the newly formed particle.

Although at least one (first) surface modifier should be dissolved along with the water insoluble substance to be reduced in size in the liquefied gas, additional (second) surface modifying agents of the same or different chemical nature may also be included in the aqueous medium. Further, during or after precipitation the fluid streams may be subjected to additional high shear forces, cavitation or turbulence by a high-pressure homogenizer to facilitate intimate contact of the particle surface and the surface modifier. Thus, in those cases where all the surface modifier is dispersed in the aqueous medium and the liquefied gas contains only the water insoluble substance, additional high shear forces, cavitation or turbulence by a high-pressure homogenizer can be exploited to facilitate the intimate contact of the particle surface and the surface modifier.

### Antisolvent Precipitation

A solution of the biologically active substance and other ingredients in a volatile solvent is mixed with an antisolvent which cannot solubilize the formulation components, and thus results in the active substance precipitated together with other ingredients. Suitable particle size distribution of precipitate is achieved by appropriate control of the conditions such as concentration, rate of mixing of the solution and anti-solvent, temperature and pressure. Solvent or antisolvent may be selected from suitable organic solvents as well as supercritical fluids, for instance, liquefied gasses or supercritical fluids such as supercritical carbon dioxide.

### Liquid or Solvent or Antisolvent Media Removal:

Excess liquid dispersing medium that contains the micronized particles, such as water or organic solvents, is removed by the process of drying. In case the solvent or anti-solvent medium is a supercritical compressed fluid, it is removed by decompression. The decompression of supercritical compressed fluid solution also results in the microparticulate formation.

### EXAMPLES

***Example I:*** Solubility of biologically active substances, particularly drug substances in lipophilic and hydrophilic solvents. The example drug, Itraconazole was found to be very poorly soluble in pharmaceutically acceptable lipophilic vehicles. Itraconazole solubility of at least 25 mg/mL in oil is required for making an oil-in-water type emulsion-formulation. The solvents mentioned in the following table were evaluated. Solubilities, determined by high performance liquid chromatography (HPLC) assay of the dissolved drug in water and several non-aqueous vehicles, are given the following table in units of mg/mL. Low solubility of this drug in either water or organic solvents renders it suitable for formulating as a stable surface modified particulate dispersion in a non-aqueous carrier system.

| **Solubility of Itraconazole (mg/mL) in some Solvents by HPLC** | |
|---|---|
| Water | 0.01 |
| Decyl oleate | 0.02 |
| Ethyl oleate | 0.04 |
| Ethyl myristate | 0.06 |
| Isopropyl myristate | 0.08 |
| Ethyl caprate | 0.10 |
| Miglyol-840 | 0.20 |
| Soybean oil | 0.25 |
| Miglyol 810 | 0.34 |
| Capric triglyceride | 0.37 |
| Ethyl alcohol | 0.54 |
| Corn oil PEG-6 ester | 1.24 |
| Propyleneglycol laurate | 1.26 |
| Ethyl caprylate | 1.57 |
| Miglyol 818 | 1.59 |
| Apricot kernel oil | 2.65 |
| Linoleic acid | 2.73 |
| PEG-200 | 3.05 |
| PEG-300 | 3.26 |
| PEG-400 | 3.33 |
| Triethyl citrate | 4.12 |
| Miglyol 812 | 5.14 |
| Glycerol triacetate | 7.55 |
| Glycerol a,a'-diacetate | 9.11 |
| 1,2 Propane diol | 12.5 |
| Glyceryl linoleate | 13.7 |
| Plurol oleique CC 497 | 16.0 |

***Example II:*** Solubilization and precipitation behavior of some drugs. As an example, 20 mg of either nifedipine or ursadiol were added into 1 mL of either a medium chain triglyceride, Miglyol 810, or to triacetin, or to benzyl benzoate. Almost all of the nifedipine that was added was observed to remain unsolubilized. Similarly ursodiol was not soluble in either Miglyol 810 or triacetin. This makes it possible to prepare compositions of nifedipine and ursodiol in these non-aqueous media as microparticulate suspension by further particle size reduction.

However, ursodiol was found to dissolve in benzyl benzoate. A portion of the ursodiol solution in benzyl benzoate was transferred to water and shaken. A mixture containing oily droplets resulted. No precipitation was seen occurring. On examining the mixture by an optical microscope under polarized light conditions surprisingly particles of regular crystalline shape and below 10 micron size were observed. This observation makes it possible to further prepare compositions of ursodiol utilizing appropriate solvent system and surface active agents so that micron-sized particle will be generated spontaneously upon addition of these compositions in aqueous media.

***Example III:*** A mixture containing itraconazole and egg-phospholipid in ethyl oleate was subjected to high-pressure homogenization in an Avestin Emulsiflex C5 homogenizer. Homogenizing pressures of up to 20,000 psi were used. The process fluid temperature was maintained at below 40°C by cooling with a heat exchanger equipped with a jacket of coolant fluid. Fine particulate suspension of volume weighted mean particle size of about 0.6 micron was produced.

***Example IV:*** Many compositions containing cyclosporine, omega-3 fatty acid, its ethyl ester or triglyceride, surfactants such as vitamin E-TPGS, Tween-20, Tween-80, Labrasol, Pluronic F68, Pluronic L44, co-surfactants such as ethanol, propylene glycol, or sodium hydroxide dissolved in either ethanol or propylene glycol were prepared. These compositions were highly concentrated and could be encapsulated in appropriate capsules. Upon adding to aqueous medium many of these compositions produced opaque milky-white suspensions containing surface-modified micron or sub-micron size solid particulate suspension. Thus these compositions can be further developed in the ingestable dosage-forms that facilitate efficient delivery of the drug substance upon mixing with aqueous media within *in-vivo* environment via surface-modified micron or sub-micron size particulate suspension.

Examples of cyclosporine and fenofibrate compositions which self-disperse into micron or sub-micron size solid particulate suspensions on addition of aqueous medium are shown in Tables I, II and III. Required amounts (in milligrams) of the ingredients were weighed into a 20-mL clear glass vial and mixed until complete dissolution was observed. Twenty milliliters water or simulated gastric fluid (SGF) or SGF mixed with water was added and gently mixed by inverting the mixture by hand several times. Milky white microparticulate suspension formed and demonstrated self-dispersibility property of the composition.

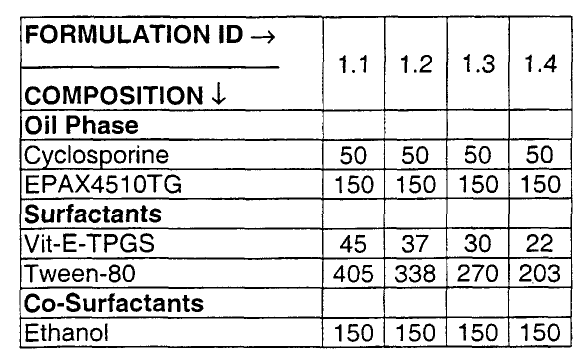

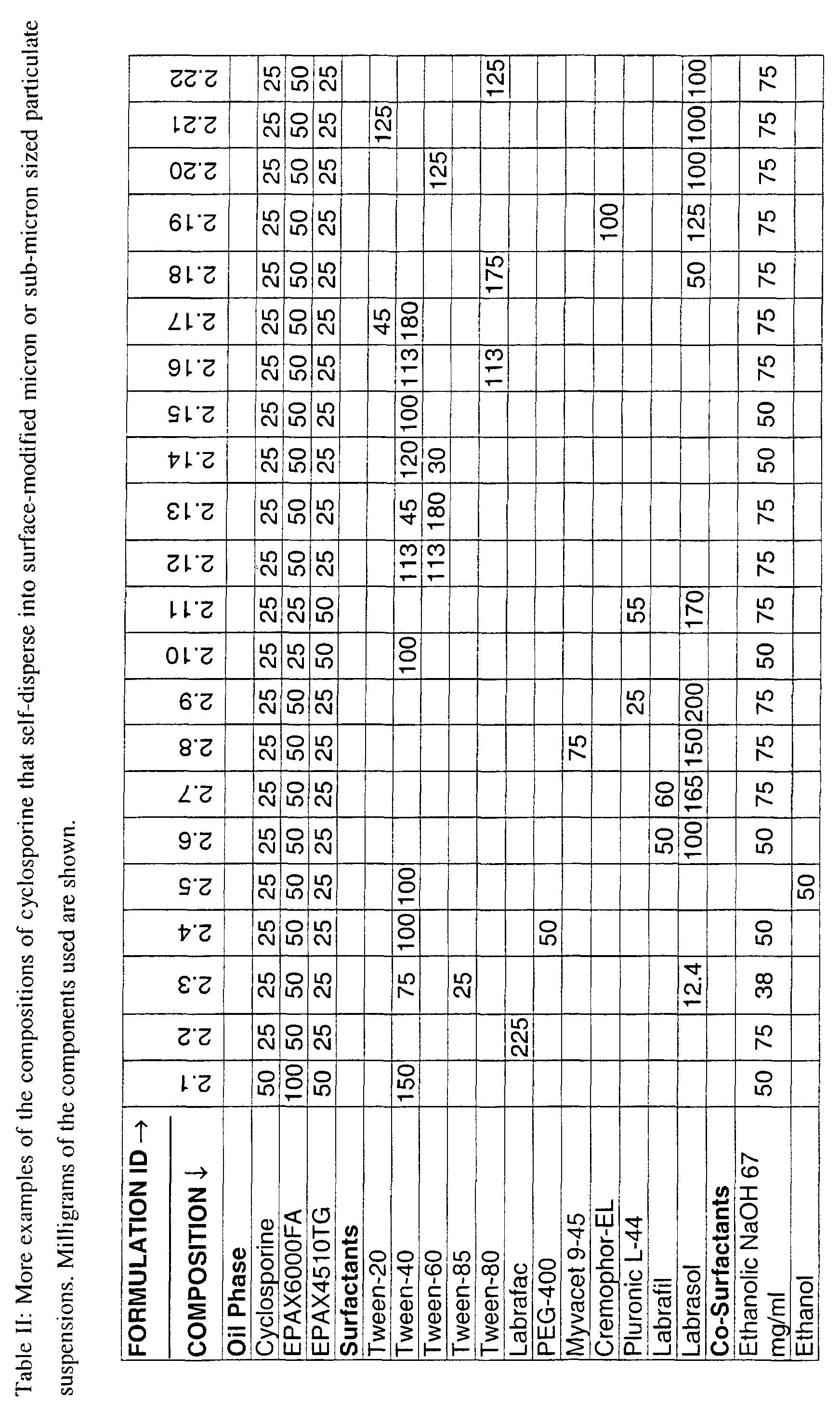

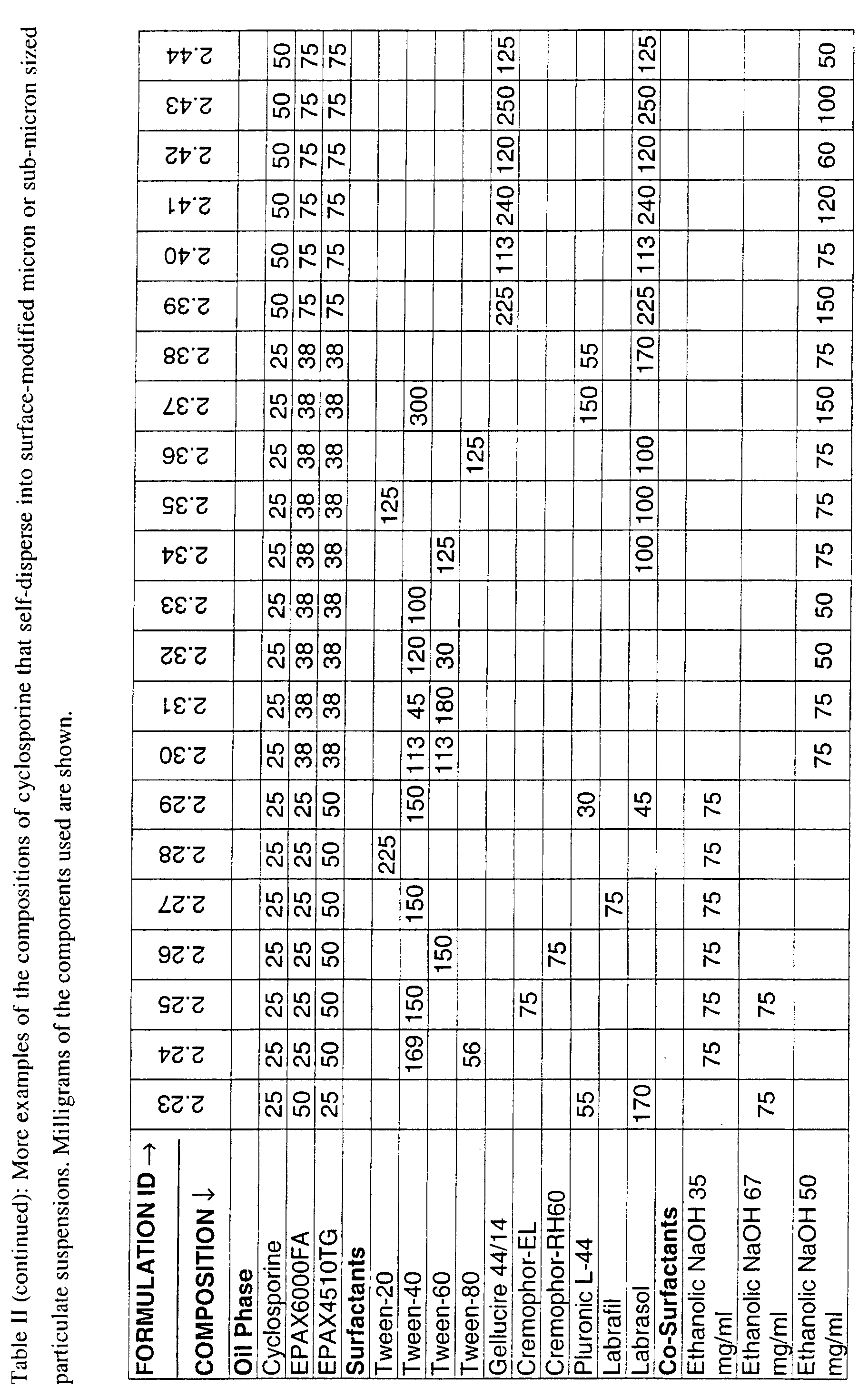

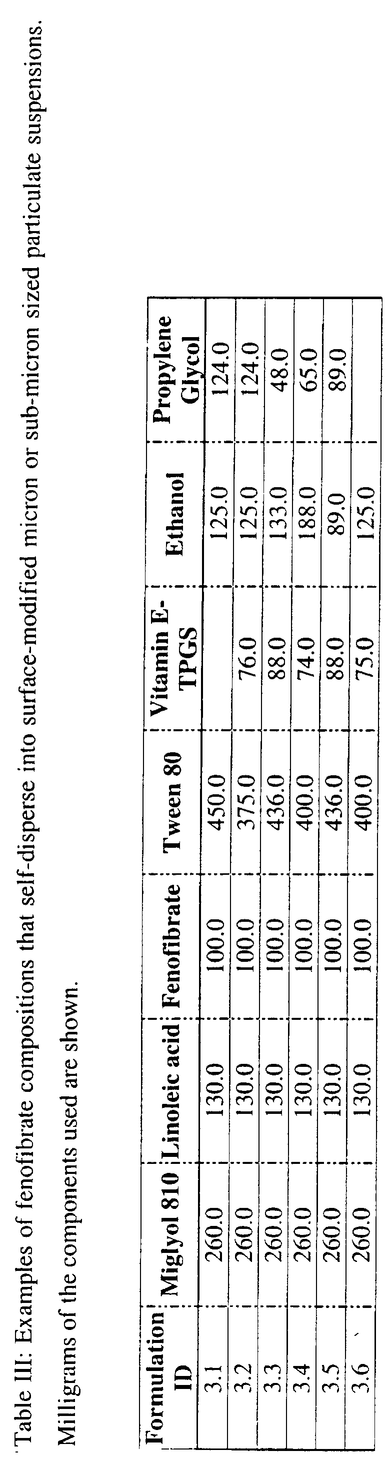

Compositions in accordance with the present invention may include additional ingredients, for example, diluents or bulking agents, anti-oxidants, preserving agents, and pH buffering agents. The above examples are illustrative but not limiting examples of compositions in accordance with the present invention.

## Claims

1. A composition comprising stable, surface modified particles of a water-insoluble biologically active substance, said particles of a mean particle size in the range of 0.01 to 10 micrometers, and dispersed in a non-aqueous carrier system comprising:
(i) a non-aqueous medium in which said biologically active substance, selected from the group consisting of nifedipine, ursodiol, budesonide, paclitaxel, camptothecin, piroxicam, itraconazole, acyclovir, fenofibrate, cyclosporine, insulin and derivatives thereof, is not soluble or is poorly soluble;
(ii) a surfactant system consisting of at least one phospholipid surfactant which is soluble in said non-aqueous medium, wherein at least a portion of said phospholipid surfactant system adsorbs to the surface of said particles; and
(iii) one or more hydrophilic substances that provide a self-dispersing property to said composition, said one or more hydrophilic substances being present in an amount of not more than 10% of the total weight of said composition;
said carrier system allowing the composition to self-disperse when added to a fluid aqueous medium to form a suspension comprising components of the non-aqueous carrier system and stable particles of the water-insoluble, biologically active substance, said particles having a size in the range of 0.01 to 10 micrometer and having associated therewith on their surface, at least a portion of the phospholipid surfactant system.

2. A composition comprising a water-insoluble biologically active substance mixed with a in a non-aqueous carrier system, said non-aqueous carrier system comprising:
(i) a non-aqueous medium in which the water-insoluble biologically active substance is soluble;
(ii) a surfactant system consisting of at least one phospholipid surfactant; and
(iii) optionally, one or more hydrophilic substances that provide a self-dispersing property to said composition, said one or more hydrophilic substances being present in an amount of not more than 10% of the total weight of said composition;
said carrier system allowing the composition to self-disperse when added to a fluid aqueous medium to form a suspension comprising components of the non-aqueous carrier system and stable particles of said water-insoluble biologically active substance, said particles having a size in the range of 0.01 to 10 micrometers and having associated therewith on their surface at least a portion of the phospholipid surfactant system.

3. The composition according to claim 1 or claim 2, wherein the non-aqueous medium is selected from the group consisting of an oil derived from animal origin; a vegetable oil; a fish oil; a fish oil free fatty acid; oleic acid; linoleic acid; a poly-unsaturated fatty acid; caprilic/capric triglyceride; caprylic/capric/linoleic triglyceride; a synthetic medium chain triglyceride having a C₈₋₁₂ fatty acid chain; propylene glycol dicaprylate/caprate; linoleic acid ethyl ester; a cholesteryl fatty acid ester, a C₁₂₋₁₈ fatty acid monoglyceride, a C₁₂₋₁₈ fatty acid diglyceride, and a C₁₂₋₁₈ fatty acid triglyceride prepared from soybean oil, almond oil, sunflower oil, olive oil, and corn oil with glycerol; a pharmaceutically acceptable monohydric alcohol; a pharmaceutically acceptable alkanol; a pharmaceutically acceptable dihydric alcohol; a pharmaceutically acceptable polyhydroxy compound; a pharmaceutically acceptable aromatic ester; benzyl beazoate; diethyl phthalate; propyl gallate; triacetin; diacetin; monoacetin; triethyl citrate; a pharmaceutically suitable hydrophobic organic solvent; a hydrofluorocarbon in the liquid state at ambient temperature and pressure; and perfiubron.

4. The composition according to claim 1 or claim 2, in which an additional surfactant component is selected from the group consisting of a natural or synthetic amphiphilic agent.

5. The composition according to claim 4, wherein the amphiphilic agent is selected from the group consisting of a phospholipid; a nonionic surfactant; a polyoxyethylene fatty alcohol ether; a sorbitan fatty acid ester; a polyoxyethylene sorbitan fatty acid ester; glycerol triacetate; a polyethylene glycol; cetyl alcohol; cetostearyl alcohol; stearyl alcohol; a poloxainer; a polaxamine; a polyoxethylene castor oil derivative; vitamin E; D-alpha-tocopheryl polyethylene glycol 1000 succinate (vitamin E TPGS); a PEG glyceryl fatty acid ester; PEG-8 glyceryl caprylate/caprate; PEG-4 glyceryl caprylate/caprate; PEG-32 glyceryl laurate; PEG-6 glyceryl mono oleate; PEG-6 glyceryl linoleate; a propylene glycol mono fatty acid ester; a propylene glycol di-fatty acid ester; propylene glycol laurate; propylene glycol caprylate/caprate; diethylene glycol monoethyl ether; transcutol; a sorbitan fatty acid ester; a monoglyceride; an acetylated mono glyceride; glycerol monooleate; glycerol mono stearate; a mono-acetylated mono glyceride; a di-acetylated monoglyceride; monoacetin; diacetin; an anionic surfactant; a fatty acid salt; a bile salt; potassium laurate; triethanolamine stearate; sodium lauryl sulfate; an alkyl polyoxyethylene sulfate; sodium alginate; clioctyl sodium sulfosuccinate; sodium carboxymethylcellulose; calcium carboxymethylcellulose; a cationic surfactant; a pharmaceutically acceptable quaternary ammonium compound; benzalkonium chloride; cetyltrimethylammonium bromide; lauryldimethylbenzylammonium chloride; PEG 1000; PEG 1500; and PEG 3400.

6. The composition according to claim 1 or claim 2, wherein the phospholipid is selected from the group consisting of a saturated phospholipid, an unsaturated phospholipid, a synthetic phospholipid, a natural phospholipid, and a combination thereof.

7. The composition according to claim 1 or claim 2, wherein the one or more hydrophilic substances is a low-molecular weight monohydric alcohol or a low-molecular weight polyhydric alcohol.

8. The composition according to claim 7, wherein the alcohol is selected from the group consisting of ethanol, a glycol, glycerol and mixtures thereof.

9. The composition according to claim 1 or claim 2, in a dosage form for peroral, parenteral, transdermal, inhalation, or ophthalmic administration of said biologically active substance.

10. The composition according to claim 1 or claim 2, which is prepared for sustained or controlled delivery of the biologically active substance.

11. The composition according to claim 1 or claim 2, wherein the fluid aqueous medium is selected from the group consisting of water, buffered water, phosphate-buffered saline, water containing a salt, water containing a soluble agent for lyoprotection, water containing a soluble agent for cryoprotection, water containing a polyhydroxy-containing compound and mixtures thereof.

12. The composition according to claim 11, wherein the buffered water is selected from the group consisting of phosphate-buffered water, citrate-buffered water, acetate-buffered water and water buffered with a pharmaceutically acceptable pH-controlling agent.

13. The composition according to claim 11, wherein the salt is a pharmaceutically acceptable salt.

14. The composition according to claim 13, wherein the salt is sodium chloride.

15. The composition according to claim 11, wherein the soluble agent for lyoprotection or cryoprotection is selected from the group consisting of dextrose, mannitol, trehalose, sucrose and sorbitol.

16. The composition according to claim 1 or claim 2, wherein the fluid aqueous medium is selected from the group consisting of a biological fluid, blood, plasma, saliva, urine, a protein-containing solution, an aqueous suspension of a protein, lymph fluid, semen, vaginal fluid, lachrymal fluid, nasal fluid, synovial fluid, cerebral fluid, cerebrospinal fluid, amniotic fluid, pancreatic fluid, pulmonary fluid, ascites fluid, fluid from a cyst, gastric fluid, intestinal fluid, a fluid removed from a patient, a diluted biological fluid, a concentrated biological fluid, and a mixture of biological fluids from one or more patients.

17. The composition according to claim 1 or claim 2, wherein the fluid aqueous medium contains one or more surface active agents.

18. The composition according to claim 1 or claim 2, contained in a capsule of hard gelatin, or soft gelatin, or starch, which capsule dissolves in the fluid aqueous medium and optionally comprises a pharmaceutically acceptable coating for controlling the release of the biologically active substance from the capsule in the fluid aqueous medium.

19. The composition according to claim 1 or claim 2, contained in a tablet, which tablet optionally comprises a pharmaceutically acceptable coating for controlling the release of the biologically active substance.

20. A process for preparing a dosage form of a water-insoluble, biologically active substance, comprising:
adding to a fluid aqueous medium a composition comprising stable particles of the water-insoluble, biologically active substance, said particles having a mean particle size in the range of 0.01 to 10 micrometers and being dispersed in a non-aqueous carrier system comprised of:
(i) a non-aqueous medium in which said biologically active substance is not soluble or is poorly soluble;
(ii) a surfactant system consisting of at least one phospholipid surfactant which is soluble in said non-aqueous medium, wherein at least a portion of which surfactant system adsorbs to the surface of said particles; and
(iii) one or more hydrophilic substances that provide a self-dispersing property to said composition, said one or more hydrophilic substances present in an amount of not more than 10% of the total weight of said composition,
said carrier system allowing the composition to self-disperse when added to a fluid aqueous medium to form a suspension comprising (i) droplets of non-aqueous medium containing particles of surface-stabilized, water-insoluble biologically active substance suspended in oily droplets of the dispersion, and (ii) particles of the water-insoluble biologically active substance migrated into the fluid aqueous medium.

## Patentansprüche

1. Zusammensetzung, die stabile, oberflächenmodifizierte Teilchen eines wasserunlöslichen, biologischen Wirkstoffs enthält, wobei die vorerwähnten Teilchen von einer mittleren Teilchengröße im Bereich von 0,01 bis 10 Mikrometer und in einem nichtwässrigen Trägersystem dispergiert sind, das folgendes aufweist:
(I) nichtwässriges Medium, in dem der vorerwähnte biologische Wirkstoff, der aus der Gruppe Nifedipin, Ursodiol, Budesonid, Paclitaxel, Camptothecin, Piroxicam, Itraconazol, Acyclovir, Fenofibrat, Cyclosporin, Insulin und Derivaten derselben gewählt wird, unlöslich oder schlecht löslich ist,
(II) oberflächenaktives Substanzsystem, das aus mindestens einem Phospholipid-Tensid besteht, welches im vorerwähnten nichtwässrigen Medium löslich ist, wobei mindestens ein Teil des vorerwähnten Phospholipid-Tensidsystems sich durch Adsorption an die Oberfläche der vorerwähnten Teilchen bindet, und
(III) eine oder mehrere hydrophile Substanzen, die der vorerwähnten Zusammensetzung die Eigenschaft der Selbstdispergierung verleihen, wobei eine oder mehrere hydrophile Substanzen in einer Menge von nicht mehr als 10 % des Gesamtgewichts der Zusammensetzung vorhanden sind,
wobei das vorerwähnte Trägersystem der Zusammensetzung ermöglicht, sich selbst zu dispergieren, wenn es einem flüssigen wässrigen Medium hinzugefügt wird, um eine Suspension zu bilden, die Komponenten des nichtwässrigen Trägersystems und stabile Teilchen des wasserunlöslichen, biologischen Wirkstoffs enthält, wobei die vorerwähnten Teilchen eine Größe im Bereich von 0,01 bis 10 Mikrometer besitzen und mit deren Oberfläche zumindest ein Teil des Phospholipid-Tensidsystems verbunden ist.

2. Zusammensetzung, die einen wasserunlöslichen, biologischen Wirkstoff, gemischt mit einem nichtwässrigen Trägersystem, enthält, **dadurch gekennzeichnet, dass** das vorerwähnte nichtwässrige Trägersystem folgendes enthält:
(I) ein nichtwässriges Medium, in dem der wasserunlösliche, biologische Wirkstoff löslich ist,
(II) ein Tensidsystem, das aus mindestens einem Phospholipid-Tensid besteht, und
(III) gegebenenfalls eine oder mehrere hydrophile Substanzen, die der vorerwähnten Zusammensetzung die Eigenschaft der Selbstdispergierung verleihen, wobei eine oder mehrere hydrophile Substanzen in einer Menge von höchstens 10 % des Gesamtgewichts der vorerwähnten Zusammensetzung vorhanden sind,
wobei das vorerwähnte Trägersystem der Zusammensetzung ermöglicht, sich selbst zu dispergieren, wenn es einem flüssigen wässrigen Medium zur Bildung einer Suspension hinzugefügt wird, die Komponenten des nichtwässrigen Trägersystems und stabile Teilchen des vorerwähnten wasserunlöslichen, biologischen Wirkstoffs enthält, wobei die vorerwähnten Teilchen eine Größe im Bereich von 0,01 bis 10 Mikrometern besitzen und mit deren Oberfläche zumindest ein Teil des Phospholipid-Tensidsystems verbunden ist.

3. Zusammensetzung nach Anspruch 1 oder Anspruch 2, **dadurch gekennzeichnet, dass** das nichtwässrige Medium aus der Gruppe gewählt wird, die aus einem Öl tierischen Ursprungs, einem pflanzlichen Öl, einem Fischöl, einer fischölfreien Fettsäure, Ölsäure, Linolsäure, einer mehrfach ungesättigten Fettsäure, Capryl/Caprin-triglyzerid, Capryl/Caprin/Linoltriglyzerid, einem synthetischen Mittelkettentriglyzerid, das eine C₈₋₁₂-Fettsäurekette besitzt, Propylenglycoldiocaprylat/caprat, Linolsäureethylester, einem Cholesterolfettsäureester, einem C₁₂₋₁₈-Fettsäuremonoglyzerid, einem C₁₂₋₁₈-Fettsäurediglyzerid und einem C₁₂₋₁₈-Fettsäuretriglyzerid besteht, welche aus Sojabohnenöl, Mandelöl, Sonnenblumenöl, Olivenöl und Maisöl mit Glycerol; aus einem pharmazeutisch akzeptablen einwertigen Alkohol; einem pharmazeutisch akzeptablen Alkanol; einem pharmazeutisch akzeptablen zweiwertigen Alkohol; einer pharmazeutisch akzeptablen Polyhydroxyverbindung; einem pharmazeutisch akzeptablen aromatischen Ester; Benzylbenzoat, Diethylphtalat; Propylgallat; Triacetin; Diacetin; Monoacetin; Triethylcitrat; einem pharmazeutisch akzeptablen geeigneten hydrophoben organischen Lösungsmittel; Fluorkohlenwasserstoff bei Raumtemperatur und -druck in flüssigem Zustand und Perfiubron abgeleitet sind.

4. Zusammensetzung nach Anspruch 1 oder Anspruch 2, bei der eine zusätzliche Tensidkomponente aus der Gruppe der natürlichen oder synthetischen amphipathischen Mittel ausgewählt ist.

5. Zusammensetzung nach Anspruch 4, **dadurch gekennzeichnet, dass** das amphipathische Mittel aus der Gruppe gewählt ist, die aus einem Phospholipid, einem nichtionischen Tensid, einem Polyoxyethylen-Fettsäureester, einem Sorbitan-Fettsäureester, einem Polyoxyethylen-Sorbitan-Fettsäureester, Glyceroltriacetat, einem Polyethylenglycol, Cetylalkohol, Cetostearylalkohol, Stearylalkohol, einem Poloxamer, einem Polaxamin, einem Polyoxyethylen-Rizinusöl-Derivat, Vitamin E, D-Alpha-Tocopherylpolyethylenglycol 1000-Succinat (Vitamin E TPGS), einem PEG-Glycerylfettsäureester, PEG-8-Glycerylcaprylat/caprat, PEG-4-Glycerycaprylat/caprat, PEG-32-Glyceryllaurat, PEG-6-Glycerylmonooleat, PEG-6-Glyceryllinoleat, einem Propylenglycol-Monofettsäureester, einem Propylenglycol-Difettsäureester, Propylenglycollaurat, Propylenglycolcaprylat/caprat, Diethylenglycolmonoethylether, Transcutol, einem Sorbitan-Fettsäureester, einem Monoglyzerid, einem acetylierten Monoglyzerid, Glycerolmonooleat, Glycerolmonostearat, einem monoacetylierten Monoglyzerid, einem diacetylierten Monoglyzerid, Monoacetin, Diacetin, einem anionischen Tensid, einem Fettsäuresalz, einem Gallensalz, Natriumlaurat, Triethanolaminstearat, Natriumlaurylsulfat, einem Alkylpolyoxyethylensulfat, Natriumalginat, Dioctylnatriumsulfosuccinat, Natriumcarboxymethylcellulose, Calciumcarboxymethylcellulose, einem kationischen Tensid, einer pharmazeutisch akzeptablen quaternären Ammoniumverbindung, Benzalkoniumchlorid, Cetyltrimethylammoniumbromid, Lauryldimethylbenzylammoniumchlorid, PEG 1000, PEG 1500 und PEG 3400 besteht.

6. Zusammensetzung nach Anspruch 1 oder Anspruch 2, **dadurch gekennzeichnet, dass** das Phospholipid aus der Gruppe aus einem gesättigten Phospholipid, einem ungesättigten Phospholipid, einem synthetischen Phospholipid, einem natürlich Phospholipid und einer Kombination derselben gewählt ist.

7. Zusammensetzung nach Anspruch 1 oder Anspruch 2, **dadurch gekennzeichnet, dass** die vorerwähnten eine oder mehreren hydrophilen Substanzen einen niedermolekularen einwertigen Alkohol oder einen niedermolekularen mehrwertigen Alkohol darstellen.

8. Zusammensetzung nach Anspruch 7, **dadurch gekennzeichnet, dass** der Alkohol aus der Gruppe aus Ethanol, einem Glycol, Glycerol und Mischungen derselben ausgewählt ist.

9. Zusammensetzung nach Anspruch 1 oder Anspruch 2, in einer Dosierform für die perorale, parenterale, transdermale, Inhalations- oder Augenadministration des vorerwähnten biologischen Wirkstoffs.

10. Zusammensetzung nach Anspruch 1 oder Anspruch 2, die für verzögerte oder kontrollierte Zufuhr des biologischen Wirkstoffs hergestellt ist.

11. Zusammensetzung nach Anspruch 1 oder Anspruch 2, **dadurch gekennzeichnet, dass** das flüssige wässrige Medium aus der Gruppe gewählt ist, die aus Wasser, wässriger Pufferlösung, phosphatgepufferter Salzlösung, ein Salz enthaltendem Wasser, Wasser, das ein lösliches Mittel zum Lyophilierschutz enthält, Wasser, das ein lösliches Mittel zum Gefrierschutz enthält, Wasser, das eine polyhydroxyhaltige Verbindung enthält, und Mischungen derselben besteht.

12. Zusammensetzung nach Anspruch 11, **dadurch gekennzeichnet, dass** die wässrige Pufferlösung aus der Gruppe gewählt ist, die aus phosphatgepuffertem Wasser, citratgepuffertem Wasser, acetatgepuffertem Wasser und Wasser, das ein pharmazeutisch akzeptables pH-Wert regulierendes Mittel enthält, besteht.

13. Zusammensetzung nach Anspruch 11, **dadurch gekennzeichnet, dass** das Salz ein pharmazeutisch akzeptables Salz ist.

14. Zusammensetzung nach Anspruch 13, **dadurch gekennzeichnet, dass** das Salz Natriumchlorid ist.

15. Zusammensetzung nach Anspruch 11, **dadurch gekennzeichnet, dass** das lösliche Mittel für den Lyophilierschutz oder den Gefrierschutz aus der Gruppe aus Dextrose, Mannitol, Trehalose, Saccharose und Sorbitol gewählt wird.

16. Zusammensetzung nach Anspruch 1 oder Anspruch 2, **dadurch gekennzeichnet, dass** das flüssige wässrige Medium aus der Gruppe gewählt ist, die aus einer biologischen Flüssigkeit, Blut, Plasma, Speichel, Urin, einer proteinhaltigen Lösung, einer wässrigen Suspension eines Proteins, Lymphflüssigkeit, Samenflüssigkeit, Vaginalflüssigkeit, Tränenflüssigkeit, Nasenflüssigkeit, Synovialflüssigkeit, Hirnflüssigkeit, Rückenmarksflüssigkeit, Fruchtwasser, Pankreasflüssigkeit, Lungenflüssigkeit, Aszitesflüssigkeit, Flüssigkeit aus einer Zyste, Magenflüssigkeit, Darmflüssigkeit, einer einem Patienten abgenommenen Flüssigkeit, einer verdünnten biologischen Flüssigkeit, einer konzentrierten biologischen Flüssigkeit und einer Mischung biologischer Flüssigkeiten von einem oder mehreren Patienten besteht.

17. Zusammensetzung nach Anspruch 1 oder Anspruch 2, **dadurch gekennzeichnet, dass** das flüssige wässrige Medium ein oder mehrere oberflächenaktive Mittel enthält.

18. Zusammensetzung nach Anspruch 1 oder Anspruch 2, die in einer Kapsel aus Hartgelatine oder Weichgelatine oder Stärke enthalten ist, wobei diese Kapsel sich im flüssigen wässrigen Medium auflöst und gegebenenfalls eine pharmazeutisch akzeptable Ummantelung zur Regulierung der Freisetzung des biologischen Wirkstoffs aus der Kapsel in das flüssige wässrige Medium enthält.

19. Zusammensetzung nach Anspruch 1 oder Anspruch 2, die in einer Tablette enthalten ist, wobei diese Tablette gegebenenfalls eine pharmazeutisch akzeptable Ummantelung zur Regulierung der Freisetzung des biologischen Wirkstoffs enthält.

20. Verfahren zur Herstellung einer Dosierform für einen wasserunlöslichen, biologischen Wirkstoff, das folgendes aufweist:
Hinzufügen einer Zusammensetzung zu einem flüssigen wässrigen Medium, wobei die Zusammensetzung stabile Teilchen des wasserunlöslichen, biologischen Wirkstoffs enthält, wobei die vorerwähnten Teilchen eine mittlere Teilchengröße im Bereich von 0,01 bis 10 Mikrometer besitzen und in einem nichtwässrigen Trägersystem dispergiert sind, welches folgendes enthält:
(I) nichtwässriges Medium, in dem der vorerwähnte biologische Wirkstoff nicht oder schwer löslich ist,
(II) Tensidsystem, das aus mindestens einem Phospholipid-Tensid besteht, das im vorerwähnten nichtwässrigen Medium löslich ist, wobei zumindest ein Teil dieses Tensidsystems sich durch Adsorption mit der Oberfläche der vorerwähnten Teilchen verbindet, und
(III) eine oder mehrere hydrophile Substanzen, die für die Eigenschaft der Selbstdispergierung für die vorerwähnte Zusammensetzung sorgen, wobei die vorerwähnten eine oder mehrere hydrophilen Substanzen in einer Menge von nicht mehr als 10 % des Gesamtgewichts der vorerwähnten Zusammensetzung vorhanden sind,
wobei das vorerwähnte Trägersystem ermöglicht, dass sich die Zusammensetzung selbst dispergiert, wenn sie einem flüssigen wässrigen Medium zur Bildung einer Suspension hinzugefügt wird, die aus folgendem besteht: (I) Tröpfchen des nichtwässrigen Mediums, das Teilchen des oberflächenstabilisierten, wasserunlöslichen, biologischen Wirkstoffs enthält, der in Öltröpfchen der Dispersion verteilt ist, und (II) Teilchen des wasserunlöslichen, biologischen Wirkstoffs, der in das flüssige wässrige Medium gewandert ist.

## Revendications

1. Composition comprenant des particules stables, à surface modifiée, d'une substance biologiquement active insoluble dans l'eau, lesdites particules, d'une taille moyenne de particules dans la plage allant de 0,01 à 10 micromètres, étant dispersées dans un système vecteur non aqueux comprenant :
(i) un milieu non aqueux dans lequel ladite substance biologiquement active, sélectionnée dans le groupe se composant de nifédipine, ursodiol, budésonide, paclitaxel, camptothécine, piroxicam, itraconazole, acyclovir, fénofibrate, cyclosporine, insuline et leurs dérivés, n'est pas soluble ou est peu soluble;
(ii) un système tensio-actif se composant d'au moins un tensio-actif phospholipidique qui est soluble dans ledit milieu non aqueux, dans lequel au moins une partie dudit système tensio-actif phospholipidique s'adsorbe à la surface desdites particules; et
(iii) une ou plusieurs substance(s) hydrophile(s) conférant une propriété d'auto-dispersion à ladite composition, ladite ou lesdites substance(s) hydrophile(s) étant présente(s) en une quantité ne dépassant pas 10% du poids total de ladite composition;
ledit système vecteur permettant à la composition de s'auto-disperser lorsqu'elle est ajoutée à un milieu liquide aqueux pour former une suspension comprenant des composants du système vecteur non aqueux et des particules stables de la substance biologiquement active insoluble dans l'eau, lesdites particules ayant une taille dans la plage allant de 0,01 à 10 micromètres et ayant, associée à leur surface, au moins une partie du système tensio-actif phospholipidique.

2. Composition comprenant une substance biologiquement active insoluble dans l'eau mélangée avec un système vecteur non aqueux, ledit système vecteur non aqueux comprenant :
(i) un milieu non aqueux dans lequel la substance biologiquement active insoluble dans l'eau est soluble;
(ii) un système tensio-actif se composant d'au moins un tensio-actif phospholipidique; et
(iii) facultativement, une ou plusieurs substances hydrophiles conférant une propriété d'auto-dispersion à ladite composition, ladite ou lesdites substance(s) hydrophile(s) étant présente(s) en une quantité ne dépassant pas 10% du poids total de ladite composition;
ledit système vecteur permettant à la composition de s'auto-disperser lorsqu'elle est ajoutée à un milieu liquide aqueux pour former une suspension comprenant des composants du système vecteur non aqueux et des particules stables de ladite substance biologiquement active insoluble dans l'eau, lesdites particules ayant une taille dans la plage allant de 0,01 à 10 micromètres et ayant, associée à leur surface, au moins une partie du système tensio-actif phospholipidique.

3. Composition selon la revendication 1 ou la revendication 2, dans laquelle le milieu non aqueux est sélectionné dans le groupe se composant d'une huile d'origine animale; d'une huile végétale; d'une huile de poisson; d'un acide gras libre d'huile de poisson; d'acide oléique; d'acide linoléique; d'un acide gras poly-insaturé; d'un triglycéride caprilique/caprique; d'un triglycéride caprylique/caprique/linoléique; d'un triglycéride synthétique à chaîne moyenne ayant une chaîne d'acide gras de C₈₋₁₂; de dicaprylate/caprate de propylène glycol; d'un éthylester d'acide linoléique; d'un ester de cholestéryl-acide gras; d'un monoglycéride d'acide gras de C₁₂₋₁₈; d'un diglycéride d'acide gras de C₁₂₋₁₈ et d'un triglycéride d'acide gras de C₁₂₋₁₈ préparé à partir d'huile de soja, d'huile d'amande, d'huile de tournesol, d'huile d'olive et d'huile de maïs avec le glycérol; d'un mono-alcool pharmaceutiquement acceptable; d'un alcanol pharmaceutiquement acceptable; d'un di-alcool pharmaceutiquement acceptable; d'un composé polyhydroxylé pharmaceutiquement acceptable; d'un ester aromatique pharmaceutiquement acceptable; d'un benzoate de benzyle; d'un phtalate de diéthyle; d'un gallate de propyle; de triacétine; de diacétine; de monoacétine; de citrate de triéthyle; d'un solvant organique hydrophobe pharmaceutiquement approprié; d'un hydrofluorocarbone à l'état liquide à température et pression ambiantes; et d'un perflubron.

4. Composition selon la revendication 1 ou la revendication 2, dans laquelle un composant tensio-actif supplémentaire est sélectionné dans le groupe se composant d'un agent amphiphile naturel ou synthétique.

5. Composition selon la revendication 4, dans laquelle l'agent amphiphile est sélectionné dans le groupe se composant d'un phospholipide; d'un tensio-actif non ionique; d'un éther de polyoxyéthylène et d'alcool gras; d'un ester de sorbitan et d'acide gras; d'un ester polyoxyéthylène de sorbitan et d'acide gras; d'un triacétate de glycérol; d'un polyéthylène glycol; d'un alcool cétylique; d'un alcool cétostéarylique; d'un alcool stéarylique; d'un poloxamère; d'une poloxamine; d'un dérivé d'huile de ricin polyoxéthyléné; de la vitamine E; du succinate de D-alpha-tocophérol-polyéthylène glycol 1000 (vitamine E TPGS); d'un PEG-ester d'acide gras et de glycéryle; d'un PEG-8 caprylate/caprate de glycéryle; d'un PEG-4 caprylate/caprate de glycéryle; d'un PEG-32 laurate de glycéryle; d'un PEG-6 mono-oléate de glycéryle; d'un PEG-6 linoléate de glycéryle; d'un mono-ester d'acide gras et de propylène glycol; d'un diester d'acide gras et de propylène glycol; d'un laurate de propylène glycol; d'un caprylate/caprate de propylène glycol; d'un mono-éthyléther de diéthylène glycol; d'un transcutol; d'un ester d'acide gras et de sorbitan; d'un monoglycéride; d'un monoglycéride acétylé; d'un mono-oléate de glycérol; d'un monostéarate de glycérol; d'un monoglycéride mono-acétylé; d'un monoglycéride diacétylé; d'une monoacétine; d'une diacétine; d'un tensio-actif anionique; d'un sel d'acide gras; d'un sel biliaire; d'un laurate de potassium; d'un stéarate de triéthanolamine; d'un laurylsulfate de sodium; d'un sulfate d'alkyle polyoxyéthylène; d'un alginate de sodium; d'un sulfosuccinate de clioctyl sodium; de la carboxyméthylcellulose sodique; de la carboxyméthylcellulose calcique; d'un tensio-actif cationique; d'un composé d'ammonium quaternaire pharmaceutiquement acceptable; d'un chlorure de benzalkonium; d'un bromure de cétyltriméthylammonium; d'un chlorure de lauryldiméthylbenzylammonium; d'un PEG 1000; d'un PEG 1500; et d'un PEG 3400.

6. Composition selon la revendication 1 ou la revendication 2, dans laquelle le phospholipide est sélectionné dans le groupe se composant d'un phospholipide saturé, d'un phospholipide insaturé, d'un phospholipide synthétique, d'un phospholipide naturel et d'une combinaison de ceux-ci.

7. Composition selon la revendication 1 ou la revendication 2, dans laquelle la ou les substance(s) hydrophile(s) est (sont) un (des) mono-alcool(s) de faible poids moléculaire ou un (des) poly-alcool(s) de faible poids moléculaire.

8. Composition selon la revendication 7, dans laquelle l'alcool est sélectionné dans le groupe se composant de l'éthanol, du glycol, du glycérol et des mélanges de ceux-ci.

9. Composition selon la revendication 1 ou la revendication 2, dans une forme posologique pour administration orale, parentérale, transdermique, par inhalation, ou ophtalmique de ladite substance biologiquement active.

10. Composition selon la revendication 1 ou la revendication 2, qui est préparée pour une libération prolongée ou contrôlée de la substance biologiquement active.

11. Composition selon la revendication 1 ou la revendication 2, dans laquelle le milieu liquide aqueux est sélectionné dans le groupe se composant d'eau, d'eau tamponnée, d'une solution saline en tampon phosphate, d'eau contenant un sel, d'eau contenant un agent soluble pour lyoprotection, d'eau contenant un agent soluble pour cryoprotection, d'eau contenant un composé polyhydroxylé et des mélanges de celles-ci.

12. Composition selon la revendication 11, dans laquelle l'eau tamponnée est sélectionnée dans le groupe se composant d'eau en tampon phosphate, d'eau en tampon citrate, d'eau en tampon acétate et d'eau tamponnée avec un agent de contrôle du pH pharmaceutiquement acceptable.

13. Composition selon la revendication 11, dans laquelle le sel est un sel pharmaceutiquement acceptable.

14. Composition selon la revendication 13, dans laquelle le sel est le chlorure de sodium.

15. Composition selon la revendication 11, dans laquelle l'agent soluble pour lyoprotection ou cryoprotection est sélectionné dans le groupe se composant de dextrose, mannitol, tréhalose, saccharose et sorbitol.

16. Composition selon la revendication 1 ou la revendication 2, dans laquelle le milieu liquide aqueux est sélectionné dans le groupe se composant de liquide biologique, sang, plasma, salive, urine, une solution protéique, une suspension aqueuse d'une protéine, lymphe, sperme, sécrétions vaginales, larmes, sécrétions nasales, liquide synovial, liquide cérébral, liquide céphalorachidien, liquide amniotique, liquide pancréatique, liquide pulmonaire, liquide d'ascite, liquide d'un kyste, liquide gastrique, liquide intestinal, liquide prélevé chez un patient, liquide biologique dilué, liquide biologique concentré, et d'un mélange de liquides biologiques d'un ou de plusieurs patients.

17. Composition selon la revendication 1 ou la revendication 2, dans laquelle le milieu liquide aqueux contient un ou plusieurs agents tensio-actifs.

18. Composition selon la revendication 1 ou la revendication 2, contenue dans une capsule de gélatine dure ou de gélatine molle ou d'amidon, laquelle capsule se dissout dans le milieu liquide aqueux et comprend facultativement un enrobage pharmaceutiquement acceptable pour contrôler la libération de la substance biologiquement active de la capsule dans le milieu liquide aqueux.

19. Composition selon la revendication 1 ou la revendication 2, contenue dans un comprimé, lequel comprimé comprend facultativement un enrobage pharmaceutiquement acceptable pour contrôler la libération de la substance biologiquement active.

20. Procédé pour préparer une forme posologique d'une substance biologiquement active insoluble dans l'eau, comprenant :
l'addition à un milieu liquide aqueux d'une composition comprenant des particules stables de la substance biologiquement active insoluble dans l'eau, lesdites particules ayant une taille moyenne dans la plage allant de 0,01 à 10 micromètres et étant dispersées dans un système vecteur non aqueux comprenant :
(i) un milieu non aqueux dans lequel ladite substance biologiquement active n'est pas soluble ou est peu soluble;
(ii) un système tensio-actif se composant d'au moins un tensio-actif phospholipidique qui est soluble dans ledit milieu non aqueux, dans lequel au moins une partie dudit système tensio-actif s'adsorbe à la surface desdites particules; et
(iii) une ou plusieurs substance(s) hydrophile(s) conférant une propriété d'auto-dispersion à ladite composition, ladite ou lesdites substance(s) hydrophile(s) étant présente(s) en une quantité ne dépassant pas 10% du poids total de ladite composition;
ledit système vecteur permettant à la composition de s'auto-disperser lorsqu'elle est ajoutée à un milieu liquide aqueux pour former une suspension comprenant (i) des gouttelettes de milieu non aqueux contenant des particules de substance biologiquement active insoluble dans l'eau, à surface stabilisée, en suspension dans des gouttelettes huileuses de la dispersion, et (ii) des particules de la substance biologiquement active insoluble dans l'eau ayant migré dans le milieu liquide aqueux.
